# EUROPÄISCHE PATENTSCHRIFT

(11) **EP 2 748 319 B1**
(45) Veröffentlichungstag und Bekanntmachung des Hinweises auf die Patenterteilung: **20.04.2016**
(21) Anmeldenummer: 13704605.8
(22) Anmeldetag: 14.02.2013
(51) Int. Cl.: C12N 15/10, C12N 15/66

(54) **VERFAHREN ZUR HERSTELLUNG EINER NUKLEINSÄURE- BIBLIOTHEK MIT MINDESTENS ZWEI BENACHBARTEN VARIABLEN CODON- TRIPLETTS**
METHOD FOR PRODUCING A NUCLEIC ACID LIBRARY HAVING AT LEAST TWO ADJACENT VARIABLE CODON TRIPLETS
PROCÉDÉ DE PRODUCTION D'UNE BIBLIOTHÈQUE D'ACIDES NUCLÉIQUES COMPRENANT AU MOINS DEUX TRIPLETS DE CODON VARIABLES VOISINS

(30) Priorität: 20.02.2012 DE 102012101347
(43) Veröffentlichungstag der Anmeldung: 02.07.2014
(73) Patentinhaber: Fuhrmann, Markus, 93142 Maxhuette-Haidhof (DE)
(72) Erfinder: Fuhrmann, Markus, 93142 Maxhuette-Haidhof (DE)
(74) Vertreter: Benninger, Johannes
(86) Internationale Anmeldenummer: PCT/EP2013/053008
(87) Internationale Veröffentlichungsnummer: WO 2013/124211

(56) Entgegenhaltungen:
- EP-A1- 1 314 783
- EP-A1- 2 110 435
- WO-A1-2004/035781
- WO-A1-2005/071077
- WO-A2-00/75368
- WO-A2-2005/059096

## Beschreibung

Die vorliegende Erfindung betrifft ein Verfahren zur Herstellung einer NukleinsäureBibliothek mit mindestens zwei benachbarten variablen Codon- Tripletts.

In der biomedizinischen Forschung werden häufig DNAs mit definierten Sequenzen benötigt, um bestimmte Zusammenhänge besser erforschen zu können. Beispielsweise werden Genbibliotheken für Antikörper benötigt, die Codon- Tripletts für unterschiedliche Aminosäuren an speziellen Schlüsselpositionen aufweisen, um optimierte Antikörper für bestimmte Zwecke identifizieren zu können. Hierbei werden häufig synthetisch hergestellte DNAs bevorzugt, die jeweils definierte Sequenzbereiche und variable Sequenzbereiche umfassen.

EP 2110435 B1 beschreibt ein Verfahren zur Herstellung einer NukleinsäureBibliothek mit einer Vielzahl von Elementen, wobei jedes Element einen ersten, einen zweiten und einen dritten Nukleotid- Abschnitt umfasst. Die Sequenz des ersten und des dritten Nukleotid- Abschnitts sind in den verschiedenen Elementen jeweils identisch, während sich die Elemente der Nukleinsäure- Bibliothek in der Sequenz des zweiten Abschnitts unterscheiden. Das Verfahren verwendet ein erstes zumindest teilweise doppelsträngiges Oligonukleotid mit einem einzelsträngigen Überhang, der die Sequenz des ersten oder dritten Nukleotid- Abschnitts umfasst. Dieses wird mit einer Oligonukleotid- Bibliothek verbunden. Diese besteht aus zweiten zumindest teilweise doppelsträngigen Oligonukleotiden, die einen komplementären Überhang aufweisen. Das Ligationsprodukt wird geschnitten und mit einer zweiten Oligonukleotid- Bibliothek zu einem zweiten Ligationsprodukt kombiniert. Diese Schritte werden so oft durchgeführt, bis der variable mittlere Teil mit der gewünschten Anzahl variabler Codon- Tripletts vollständig synthetisiert ist. Nachteilig ist insbesondere, dass in jedem Schritt die Verlängerung nur um jeweils ein Codon- Triplett erfolgt. Zudem muss jedes erste Ligationsprodukt aus der ersten Oligonukleotid- Bibliothek mit jedem Oligonukleotid der zweiten Oligonukleotid- Bibliothek kombiniert werden. Dies bedingt einen hohen apparativen und rechnerischen Aufwand.

Aufgabe der Erfindung ist es, ein einfaches, schnelles und kostengünstiges Verfahren zur Herstellung von Nukleinsäure- Bibliotheken mit mindestens zwei benachbarten variablen Codon- Tripletts bereitzustellen, bei dem der apparative Aufwand gegenüber herkömmlichen Methoden verringert ist.

Die obige Aufgabe wird durch ein Verfahren gelöst, das die Merkmale in dem Patentanspruch 1 umfasst. Weitere vorteilhafte Ausgestaltungen werden durch die Unteransprüche beschrieben.

Die Erfindung betrifft ein Verfahren zur Herstellung einer DNA- Sequenz für eine Nukleinsäure- Bibliothek mit einer Vielzahl von Elementen. Die DNA- Sequenz umfasst jeweils mindestens drei Bereiche. Der erste und der dritte Bereich weisen jeweils eine definierte Sequenz auf, die in allen Elementen der Nukleinsäure- Bibliothek jeweils identisch ist. Die Erfindung bezieht sich insbesondere auf Nukleinsäure- Bibliotheken, bei denen im mittleren zweiten Bereich jeweils mindestens zwei benachbarte variable Codon-Tripletts angeordnet sind. Dies ist insbesondere wichtig, um den Austausch benachbarter Aminosäuren, beispielsweise im aktiven Zentrum von Proteinen, gezielt untersuchen zu können.

Das Verfahren wird mit mindestens zwei Oligonukleotid- Bibliotheken und mindestens zwei weitgehend komplementären einzelsträngigen Oligonukleotiden oder Komplementär- Oligonukleotid- Bibliotheken durchgeführt, die die nachfolgend beschriebenen Eigenschaften aufweisen:
Eine erste Oligonukleotid- Bibliothek besteht aus Oligonukleotiden mit jeweils einer Teilsequenz, die dem definierten ersten Bereich der Nukleinsäure- Bibliothek entspricht. Danach ist mindestens ein erstes variables Codon- Triplett und daran anschließend ein erster Teilbereich eines Spacer- Elementes und ein definierter 3' Überhang angeordnet.

Ein erstes komplementäres Oligonukleotid ist zumindest komplementär zu den Oligonukleotiden der ersten Oligonukleotid- Bibliothek aufgebaut. Insbesondere weist es eine Teilsequenz auf, die komplementär zum ersten Teilbereich des Spacer- Elementes der ersten Oligonukleotid- Bibliothek ausgebildet ist. Weiterhin umfasst das erste komplementäre Oligonukleotid mindestens ein erstes weitgehend komplementärvariables Codon- Triplett und eine Teilsequenz, die komplementär zum definierten ersten Bereich der Oligonukleotide der ersten Oligonukleotid- Bibliothek aufgebaut ist. Das komplementär- variable Codon- Triplett wird vorzugsweise durch drei so genannte "Universal- Basen" oder durch ein Gemisch aus allen vier kanonischen Nukleotiden gebildet. Unter "Universal- Basen" versteht man Nukleotide, die jede der vier üblichen Nukleotide Adenin, Cytosin, Guanin und Thymin ersetzen können, ohne benachbarte Basenpaar-Wechselwirkungen signifikant zu destabilisieren oder deren biochemische Wirkungsweise im modifizierten Oligonukleotid zu stören. Oligonukleotide mit einem universellen Nukleotid können problemlos als Primer bei der DNA- Sequenzierung etc. eingesetzt werden und stören auch nicht bei enzymatischen Reaktionen mit Polynukleotidkinase, DNA- Ligase und anderen Enzymen. Solche "Universal- Basen" sind beispielsweise bekannte Analoga zu den kanonischen Basen der natürlichen Nukleinsäuren, die mit allen natürlichen Basen aus DNA und RNA komplementäre Basenpaarungen ausbilden können. Inzwischen wurden auch schon synthetisch "Universal- Basen" hergestellt, die mit den Nukleotiden dA, dC, dG oder dT Wasserstoffbrückenbindungen ausbilden, ohne die Watson- Crick- dsDNA- Struktur zu destabilisieren. Zu den inzwischen bekannten "Universal- Basen" zählen Nukleoside des 3-Methyl-7-Propinylisocarbostyril (PIM), 3-Methylisocarbostyril (MICS), 5-Methylisocarbostyril (5MICS), 3-Nitropyrrol, 5-Nitroindol und 2'-Desoxyinosine (dl).

Eine zweite Oligonukleotid- Bibliothek umfasst Oligonukleotide mit einer Teilsequenz, die den zweiten Teilbereich des Spacers bildet, mindestens einem zweiten variablen Codon- Triplett und eine Teilsequenz, die dem definierten zweiten Bereich der Nukleinsäure- Bibliothek entspricht. Ein zweites komplementäres Oligonukleotid ist weitgehend komplementär zu den Oligonukleotiden der zweiten Oligonukleotid- Bibliothek ausgebildet, mit jeweils einer Teilsequenz, die komplementär zum zweiten Bereich der Oligonukleotide der zweiten Oligonukleotid- Bibliothek ausgebildet ist, mindestens einem zweiten komplementär- variablen Codon- Triplett, einer Teilsequenz, die komplementär zum zweiten Teilbereich des Spacers der Oligonukleotide der weiteren Oligonukleotid-Bibliothek ausgebildet ist und einen daran anschließenden definierten 3' Überhang.

Anstelle der beschriebenen Oligonukleotid- Bibliotheken und entsprechenden weitgehend komplementären Oligonukleotiden können auch eine erste Oligonukleotid-Bibliothek und ein zweites komplementäres Oligonukleotid ohne 3' Überhang verwendet werden. Stattdessen werden zwei Oligonukleotide und zwei Oligonukleotid- Bibliotheken verwendet, wobei das erste komplementäre Oligonukleotid und die zweite Oligonukleotid-Bibliothek jeweils einen 5' Überhang aufweisen.

Gemäß einer bevorzugten Ausführungsform der Erfindung umfassen die definierten 3' Überhänge der Oligonukleotide der ersten Oligonukleotid- Bibliothek und des zweiten komplementären Oligonukleotids bzw. die definierten 5' Überhänge der Oligonukleotide der zweiten Oligonukleotid- Bibliothek und des ersten komplementären Oligonukleotids jeweils dieselbe Anzahl von Nukleotiden und sind zueinander zumindest weitgehend komplementär.

Zur Herstellung der gewünschten Nukleinsäure mit mindestens zwei benachbarten variablen Codon- Tripletts werden folgende Verfahrensschritte durchgeführt: Zuerst hybridisiert man die Oligonukleotide der ersten Oligonukleotid- Bibliothek jeweils mit dem weitgehend komplementären ersten Oligonukleotid und die Oligonukleotide der zweiten Oligonukleotid- Bibliothek mit dem weitgehend komplementären zweiten Oligonukleotid. Dabei entstehen jeweils erste und zweite doppelsträngige DNA- Bibliotheken. Diese werden miteinander über die komplementären Überhänge der Oligonukleotide der ersten Oligonukleotid- Bibliothek und des zweiten komplementären Oligonukleotids durch Ligation verbunden, wobei eine Bibliothek aus doppelsträngigen DNA-Ligationsprodukten entsteht. Die DNA- Ligationsprodukte umfassen jeweils folgende Teilbereiche: definierter ersten Bereich der Nukleinsäure- Bibliothek, mindestens ein erstes variables Codon- Triplett, Spacer- Sequenz, mindestens ein zweites variables Codon- Triplett und definierter zweiter Bereich der Nukleinsäure- Bibliothek. Durch Entfernen der Spacer- Sequenz aus den doppelsträngigen DNA- Ligationsprodukten wird die Bibliothek aus fertigen, doppelsträngige DNA- Produkten gebildet. Diese umfassen jeweils folgende Teilbereiche: definierter ersten Bereich der Nukleinsäure- Bibliothek, mindestens ein erstes variables Codon- Triplett, mindestens ein zweites variables Codon-Triplett und definierter zweiter Bereich der Nukleinsäure- Bibliothek.

Gemäß einer weiteren Ausführungsform der Erfindung werden die oben beschriebenen Oligonukleotide einer ersten Oligonukleotid- Bibliothek jeweils mit entsprechend weitgehend komplementären Oligonukleotiden einer ersten Komplementär-Oligonukleotid- Bibliothek hybridisiert, wobei die Oligonukleotide der ersten Komplementär- Oligonukleotid- Bibliothek jeweils folgende Teilsequenzen aufweisen: erste Teilsequenz, die komplementär zum ersten Teilbereich des Spacer- Elementes der ersten Oligonukleotid- Bibliothek ausgebildet ist, komplementäres Codon- Triplett zu einem der Oligonukleotide aus der ersten Oligonukleotid- Bibliothek und eine Teilsequenz, die komplementär zum definierten ersten Bereich der Oligonukleotide der ersten Oligonukleotid- Bibliothek aufgebaut ist. Zu jedem Oligonukleotid der ersten Oligonukleotid- Bibliothek gibt es in der ersten Komplementär- Oligonukleotid- Bibliothek ein weitgehend komplementäres Oligonukleotid. Die jeweiligen Oligonukleotide werden aufgrund des oben beschriebenen 3' oder 5' Überhangs jeweils nur als weitgehend komplementär bezeichnet. Wie bereits beschrieben wurde, weisen die Oligonukleotide der ersten Oligonukleotid- Bibliothek vorzugsweise einen komplementären 3' Überhang gegenüber den jeweils komplementären Oligonukleotiden der zweiten Komplementär-Oligonukleotid- Bibliothek auf. Alternativ weisen die Oligonukleotide der ersten Komplementär- Oligonukleotid- Bibliothek einen 5' Überhang gegenüber den Oligonukleotiden der ersten Oligonukleotid- Bibliothek auf.

Bei dieser Ausführungsform werden also jeweils zwei, bis auf die Überhänge komplementäre Oligonukleotide miteinander hybridisiert. Dies ist insbesondere vorteilhaft, wenn nur wenige unterschiedliche Codon- Tripletts getestet werden sollen. Die jeweils hybridisierten Oligonukleotide bilden zusammen die erste doppelsträngige DNA-Bibliothek. Anstelle des so genannten zweiten komplementären Oligonukleotids kann analog eine zweite Komplementär- Oligonukleotid- Bibliothek verwendet werden.

Gemäß einer Ausführungsform der Erfindung weisen die Oligonukleotide der ersten Oligonukleotid- Bibliothek und das zweite komplementäre Oligonukleotid jeweils definierte, zueinander komplenientäre 3' Überhänge auf und das erste komplementäre Oligonukleotid und die Oligonukleotide der zweiten Oligonukleotid- Bibliothek weisen ebenfalls jeweils definierte 3' Überhänge auf. Vorzugsweise sind diese 3' Überhänge unterschiedlich lang und nicht komplementär zueinander ausgebildet. Insbesondere sind die 3' Überhänge des ersten komplementären Oligonukleotids und die 3' Überhänge der Oligonukleotide der zweiten Oligonukleotid- Bibliothek jeweils unterschiedlich lang und nicht komplementär zu den definierten 3' Überhängen der Oligonukleotide der ersten Oligonukleotid- Bibliothek und des zweiten komplementären Oligonukleotids. Die unterschiedliche Ausbildung der 3' Überhänge der Oligonukleotide bzw. Oligonukleotid-Bibliotheken gewährleistet eine korrekte Ausrichtung derselben bei der Hybridisierung und anschließender Ligation.

Gemäß einer weiteren Ausführungsform der Erfindung weisen die Oligonukleotide jeweils definierte 5' Überhänge auf. Vorzugsweise sind diese 5' Überhänge der Oligonukleotide der zweiten Oligonukleotid- Bibliothek und des ersten komplementären Oligonukleotids gleich lang und komplementär zueinander ausgebildet, während die 5' Überhänge des zweiten komplementären Oligonukleotids und die 5' Überhänge der Oligonukleotide der ersten Oligonukleotid- Bibliothek jeweils unterschiedlich lang und nicht komplementär sind. Die unterschiedliche Ausbildung der 5' Überhänge der Oligonukleotide bzw. Oligonukleotid- Bibliotheken gewährleistet eine korrekte Ausrichtung derselben bei der Hybridisierung und anschließender Ligation.

Die Spacer- Sequenz umfasst mindestens eine Erkennungssequenz für mindestens eine Typ IIS Restriktionsendonuklease. Gemäß einer bevorzugten Ausführungsform umfasst die Spacer- Sequenz zwei Erkennungssequenzen für mindestens eine, vorzugsweise zwei Typ IIS Restriktionsendonukleasen. Typ IIS Restriktionsendonukleasen erkennen die zu schneidende DNA an der jeweils für sie spezifischen Erkennungssequenz und schneiden die DNA an einer Restriktionsstelle in einem definierten Abstand zur Erkennungssequenz. Die Spacer- Sequenz ist derart gewählt, dass die mindestens eine Typ IIS Restriktionsendonuklease das doppelsträngige DNA- Ligationsprodukt 3' nach dem dritten Nukleotid des ersten variablen Codon-Tripletts und / oder 5' vor dem ersten Nukleotid des zweiten variablen Codon- Tripletts schneidet. Somit kann die Spacer- Sequenz einfach aus dem DNA- Ligationsprodukt herausgeschnitten werden.

Gemäß einer Ausführungsform wird eine Typ IIS Restriktionsendonuklease verwendet, die die DNA "blunt", d.h. ohne Überhänge schneidet. Nach Religation des geschnittenen Ligationsproduktes erhält man eine DNA mit folgender Sequenz: definierter ersten Bereich der Nukleinsäure- Bibliothek, mindestens ein erstes variables Codon-Triplett, mindestens ein zweites variables Codon- Triplett und definierter zweiter Bereich der Nukleinsäure- Bibliothek. Verwendet man dagegen eine Typ IIS Restriktionsendonuklease, die beim Schneiden 3' oder 5' Überhänge erzeugt, dann müssen diese Überhänge erst durch Standardmethoden zu glatten Enden aufgefüllt oder abgebaut werden. Hierfür können beispielsweise Klenow Polymerase, T4 DNA Polymerase etc. verwendet werden. Nach Religation des geschnittenen und geglätteten Ligationsproduktes erhält man eine DNA mit oben beschriebener Sequenz.

Weiterhin kann vorgesehen sein, dass ein Oligonukleotid der ersten Oligonukleotid-Bibliothek jeweils zwei benachbarte oder weitgehend benachbarte variable Codon-Tripletts und / oder ein Oligonukleotid der zweiten Oligonukleotid- Bibliothek ebenfalls jeweils zwei benachbarte oder weitgehend benachbarte variable Codon- Tripletts aufweist. Dementsprechend weisen das erste und / oder das zweite komplementäre Oligonukleotid jeweils zwei benachbarte oder weitgehend benachbarte komplementärvariable Codon- Tripletts aus jeweils drei "Universal- Basen" auf. Wie bereits oben beschrieben wurde, können anstelle des ersten und / oder zweiten komplementären Oligonukleotids auch jeweils Komplementär- Oligonukleotid- Bibliotheken verwendet werden. Nach dem oben beschriebenen Verfahren kann nunmehr eine NukleinsäureSequenz mit jeweils drei oder vier benachbarten oder weitgehend benachbarten variablen Codon- Tripletts konstruiert werden.

Weitere Abwandlungen bei der Wahl der Oligonukleotide sind möglich, die jeweils so konstruiert sind, das nach Hybridisierung und Ligation das entsprechende doppelsträngige DNA- Ligationsprodukt entsteht. Beispielsweise kann die erste Oligonukleotid- Bibliothek aus jeweils zwei Oligonukleotiden bestehen, wobei das erste Oligonukleotid folgende Teilsequenzen aufweist: 5'- definierter erster Bereich D1 - erstes variables Codon- Triplett Tv₁₋₁ₓ und wobei das zweite Oligonukleotid folgende Teilsequenzen aufweist: zweites variables Codon- Triplett Tv_{1-1y} - erster Teilbereich S1 eines Spacers S und definierter 3'weitgehend komplementärer Überhang U1. Das erste komplementäre Oligonukleotid oder die erste Komplementär- Oligonukleotid- Bibliothek weist dementsprechend folgende Teilsequenzen auf: 5'- komplementäre Teilsequenz - zweites weitgehend komplementär variables Codon- Triplett - erstes weitgehend komplementär variables Codon- Triplett - zur definierten Sequenz komplementäre Sequenz - definierter 3' Überhang. Nach Hybridisierung und Ligation der drei Oligonukleotide entstehen dementsprechend DNA- Ligationsprodukte mit der Sequenz: definierter erster Bereich - erstes variables Codon- Triplett - zweites variables Codon-Triplett - erster Teilbereich eines Spacers. Die beschriebenen Verfahrensschritte werden vorzugsweise nicht mit linearen DNA Fragmenten, sondern jeweils in einem geeigneten Klonierungsvektor durchgeführt. Insbesondere wird die Bibliothek aus doppelsträngigem DNA- Ligationsprodukt, welches zwischen den mindestens zwei variablen Codon-Tripletts noch die Spacer- Sequenz enthält, über die definierten 3' Überhänge des ersten komplementären Oligonukleotids und die definierten 3' Überhänge der zweiten Oligonukleotid- Bibliothek durch Ligation in einen entsprechend vorbereiteten Klonierungsvektor integriert. Die dabei entstandenen Ligationsprodukt- Vektoren werden mit mindestens einer Typ IIS Restriktionsendonuklease behandelt, wobei die Spacer-Sequenz herausgeschnitten wird. Die Bibliothek aus geschnittenem Ligationsprodukt-Vektor wird anschließend durch Religation zu einer Produkt- Vektor- Bibliothek recyclisiert.

Durch Kombinationen mehrerer entsprechend konstruierter Bibliotheken von ersten, zweiten usw. Produkt- Vektoren mit Sequenzen für weitere Spacer Elemente anstelle des definierten ersten oder zweiten Bereichs der Nukleinsäure- Bibliothek, können somit Nukleinsäure- Sequenzen mit eine beliebigen Anzahl nebeneinander angeordneter variabler Codon- Tripletts erzeugt werden. Gemäß einer Ausführungsform umfasst eine erste Bibliothek von Produktvektor mit einem ersten variablen Nukleinsäure- Bereich mindestens zwei unterschiedliche Selektionsmarker. Eine zweite Bibliothek von Produktvektor mit einem zweiten variablen Nukleinsäure- Bereich umfasst mindestens zwei weitere unterschiedliche Selektionsmarker. Die zwei Produkt- Vektor- Bibliotheken werden jeweils an zwei Stellen mittels geeigneter Restriktionsendonukleasen geschnitten und die dabei entstandenen Fragmente werden miteinander durch Religation neu kombiniert. Die dabei entstehenden neue Produkt- Vektoren weisen beispielsweise zweimal den ersten variablen Nukleinsäure- Bereich, zweimal den zweiten variablen Nukleinsäure- Bereich, die Kombination aus Nukleinsäure- Bereich eins und Nukleinsäure- Bereich zwei oder die Kombination aus Nukleinsäure- Bereich zwei und Nukleinsäure- Bereich eins auf. Die Produkt- Vektoren mit der gewünschten Nukleinsäure- Kombination können durch gezielte Doppel- Selektion anhand der Selektionsmarker identifiziert werden.

Die Vorteile des erfindungsgemäßen Verfahrens bestehen insbesondere darin, dass dieses gut automatisierbar ist. Die Anzahl der notwendigen Verfahrensschritte gegenüber dem oben beschriebenen Verfahren nach dem Stand der Technik ist wesentlich reduziert. Zudem werden weniger Oligonukleotide benötigt, was wiederum die Kosten des Verfahrens verringert. Im beschriebenen Verfahren werden jeweils Bibliotheken aus hybridisierten Oligonukleotiden miteinander verknüpft, die jeweils mindestens einen Überhang aufweisen, der nach dem Entfernen des mittleren Spacer- Bereichs nicht mehr im Nukleinsäurebibliothek- Produkt zu finden ist. D.h. die den Überhang bildenden Teile der Oligonukleotid- Einzelstränge werden nachträglich entfernt und sind somit nicht Bestandteil des Endprodukts. Aus diesem Grund können für die Hybridisierungs- und Ligationsreaktion optimierte Überhänge verwendet werden. Insbesondere müssen die Überhänge nicht variabel entsprechend der gewünschten Mutation gestaltet werden. Dies vermindert den Verfahrensaufwand und verbessert die Qualität des entstehenden Endproduktes.

Bei dem Verfahren werden für die Herstellung von vier aufeinander folgenden Positionen mit einer Mutationsrate von 20 Varianten insgesamt (4-20 + 2) = 82 Oligonukleotide als Gemische bereitzustellen. Somit werden bei dem Verfahren gegenüber herkömmlich bekannten Verfahren weniger Ausgangs- Oligonukleotide benötigt, wodurch das Verfahren wesentlich kostengünstiger ist. Weiterhin ist der Arbeits- bzw. Pipettieraufwand sowie Zeitaufwand deutlich reduziert. Weiterhin können die Verknüpfungsreaktionen im beschriebenen Verfahren parallel durchgeführt werden, so dass mehrere Prozesse gleichzeitig ausgeführt werden können. Nur die Entfernungsreaktionen sind sequentiell. Diese Reaktionen sind unter Annahme sättigend hoher Enzym- und Koenzymkonzentrationen (quasi) unimolekular und damit nur von der Konzentration des Substrats abhängig. Das Substrat kann nach der Verknüpfungsreaktion durch Klonierung nahezu beliebig vervielfältigt werden.

Im Folgenden sollen Ausführungsbeispiele die Erfindung und ihre Vorteile anhand der beigefügten Figuren näher erläutern. Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind.
Figur 1 bis Figur 11 zeigen die Herstellung einer Nukleinsäure- Bibliothek mit zwei benachbarten variablen Codon- Tripletts.
Figur 12 bis Figur 16 zeigen die Herstellung einer Nukleinsäure- Bibliothek mit drei benachbarten variablen Codon- Tripletts.
Figuren 17 bis 21 zeigen die Herstellung einer Nukleinsäure- Bibliothek mit vier benachbarten variablen Codon- Tripletts.
Figuren 22 bis 24 zeigen die Herstellung von Nukleinsäure- Bibliotheken mit einer Mehrzahl an benachbarten variablen Codon- Tripletts.
Figur 25 zeigt eine weitere Ausführungsform der beim erfindungsgemäßen Verfahren verwendeten Oligonukleotide.
Figur 26 zeigt eine weitere Ausführungsform der beim erfindungsgemäßen Verfahren verwendeten Oligonukleotide.

Für gleiche oder gleich wirkende Elemente der Erfindung werden identische Bezugszeichen verwendet. Ferner werden der Übersicht halber nur Bezugszeichen in den einzelnen Figuren dargestellt, die für die Beschreibung der jeweiligen Figur erforderlich sind. Die dargestellten Ausführungsformen stellen lediglich Beispiele dar, wie das erfindungsgemäße Verfahren ausgestaltet sein kann und stellen keine abschließende Begrenzung dar.

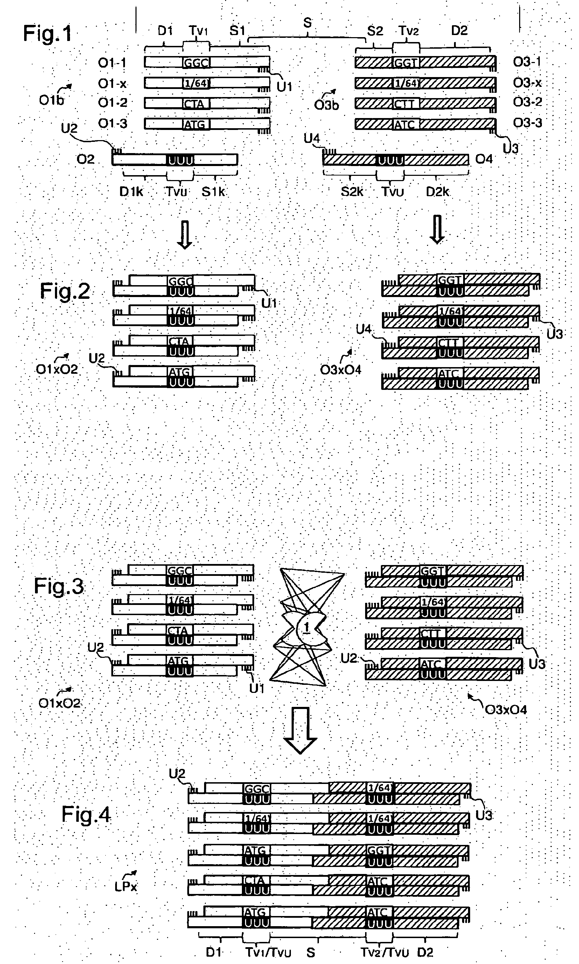

Figur 1 bis Figur 11 zeigen die Herstellung einer Nukleinsäure- Bibliothek mit zwei benachbarten variablen Codon- Tripletts T_{V1} und T_{V2}. Figur 1 zeigt die unterschiedlichen Oligonukleotide O2 und 04 bzw. Oligonukleotid- Bibliotheken O1b und O3b, die für das erfindungsgemäße Verfahren verwendet werden. Eine erste Oligonukleotid- Bibliothek O1b umfasst erste Oligonukleotide O1-x mit jeweils vier definierten Teilbereichen. Eine erste Teilsequenz D1 entspricht dem definierten ersten Bereich der NukleinsäureBibliothek. Daran anschließend findet sich ein erstes variables Codon- Triplett T_{V1}. Abhängig davon, welche unterschiedlichen Aminosäuren exprimiert werden sollen, werden entsprechend viele unterschiedliche Oligonukleotide O1-x mit unterschiedlichen Codon- Tripletts verwendet. Im Extremfall werden vierundsechzig unterschiedlichen Oligonukleotide O1-x verwendet, um das gesamt mögliche Spektrum an Aminosäuren bzw. vorzeitigen Abbrüchen der Proteinkette durch Stop- Codons abzudecken. Anschließend an das variable Codon- Triplett T_{V1} ist eine erste Teilsequenz S1 eines Spacers S angeordnet. Zudem weisen die Oligonukleotide O1-x der ersten Oligonukleotid- Bibliothek O1b einen definierten 3' Überhang U1 aus fünf Nukleotiden auf.

Das zweite Oligonukleotid O2 ist weitgehend komplementär zu den Oligonukleotiden O1-x der ersten Oligonukleotid- Bibliothek O1b aufgebaut. Es umfasst eine Teilsequenz S1k, die komplementär zur ersten Teilsequenz S1 des Spacers S der ersten Oligonukleotide O1-x aufgebaut ist. Daran schließt sich ein erstes variables Codon-Triplett Tvu an, das drei "Universal- Basen" (vgl. Beschreibung) umfasst und eine Teilsequenz D1 k, die komplementär zum ersten Bereich D1 der ersten Oligonukleotide O1-x ausgebildet ist. Weiterhin weist das zweite Oligonukleotid O2 einen 4er Nukleotid-Überhang U2 am 3' Ende auf. Dieser entspricht vorzugsweise einem Überhang, der auch beim Schneiden durch bestimmte Restriktionsenzyme entstehen würde.

Eine zweite Oligonukleotid- Bibliothek O3b umfasst dritte Oligonukleotide O3-x mit jeweils vier definierten Teilbereichen. Eine erste Teilsequenz bildet die zweite Teilsequenz S2 des Spacers S. Daran anschließend befindet sich ein zweites variables Codon- Triplett T_{V2} und eine Teilsequenz, die dem definierten zweiten Bereich D2 der NukleinsäureBibliothek entspricht. Wie auch im Fall der Oligonukleotide O1-x der ersten Oligonukleotid- Bibliothek O1b wird eine Reihe von unterschiedlichen dritten Oligonukleotiden O3-x verwendet, je nachdem, welche Aminosäuren exprimiert werden sollen. Weiterhin weisen die Oligonukleotide O3-x der zweiten Oligonukleotid- Bibliothek O3b einen 3er Nukleotid- Überhang U3 am 3' Ende auf. Dieser entspricht vorzugsweise einem Überhang, der auch beim Schneiden durch bestimmte Restriktionsenzyme entstehen würde.

Weiterhin ist ein viertes Oligonukleotid 04 dargestellt, das weitgehend komplementär zu den Oligonukleotiden O3-x der zweiten Oligonukleotid- Bibliothek O3b ausgebildet ist. Es umfasst eine Teilsequenz D2k, die komplementär zum zweiten Bereich D2 der dritten Oligonukleotide O3-x ausgebildet ist. Daran schließt sich ein zweites variables Codon- Triplett Tvu, das drei "Universal- Basen" umfasst, und eine Teilsequenz S2k, die komplementär zur zweiten Teilsequenz S2 des Spacers S des dritten Oligonukleotids O3 ausgebildet ist. Weiterhin weist das Oligonukleotid 04 einen 3' Überhang U4 aus fünf Nukleotiden auf, der komplementär zu den 3' Überhangen der ersten Oligonukleotide O1-x ausgebildet ist.

In einem ersten Verfahrensschritt werden die Oligonukleotide O1-1, O1-2, O1-3 bzw. O1-x der ersten Oligonukleotid- Bibliothek O1b jeweils mit dem zweiten Oligonukleotid O2 hybridisiert, indem ein Oligonukleotid 1-x - Oligonukleotid 2 - Gemisch zuerst auf 95°C erhitzt und anschließend langsam wieder abgekühlt wird. Entsprechend werden die dritten Oligonukleotide O3-1, O3-2, O3-3 bzw. O3-x jeweils mit dem vierten Oligonukleotid 04 hybridisiert. Die dabei entstehenden doppelsträngigen O1 xO2 bzw. O3xO4 - DNA- Produkt- Bibliotheken sind schematisch in Figur 2 dargestellt.

In einem zweiten Verfahrensschritt (vgl. Figur 3) werden die Oligonukleotid 1-x - Oligonukleotid 2 - DNAs O1xO2 mit den Oligonukleotid 3-x - Oligonukleotid 4- DNAs O3xO4 über die komplementären Überhänge U1 und U4 der Oligonukleotide 1 und 4 mit einer Ligase 1 verbunden. Einige der dabei entstehenden doppelsträngigen Ligationsprodukte LPx sind in Figur 4 dargestellt. Die Ligationsprodukte LPx umfassen folgende Teilbereiche: definierter erster Bereich D1 der Nukleinsäure- Bibliothek, erstes variables Codon- Triplett T_{V1}, Spacer- Sequenz S, zweites variables Codon- Triplett T_{V2} und definierter zweiter Bereich D2 der Nukleinsäure- Bibliothek.

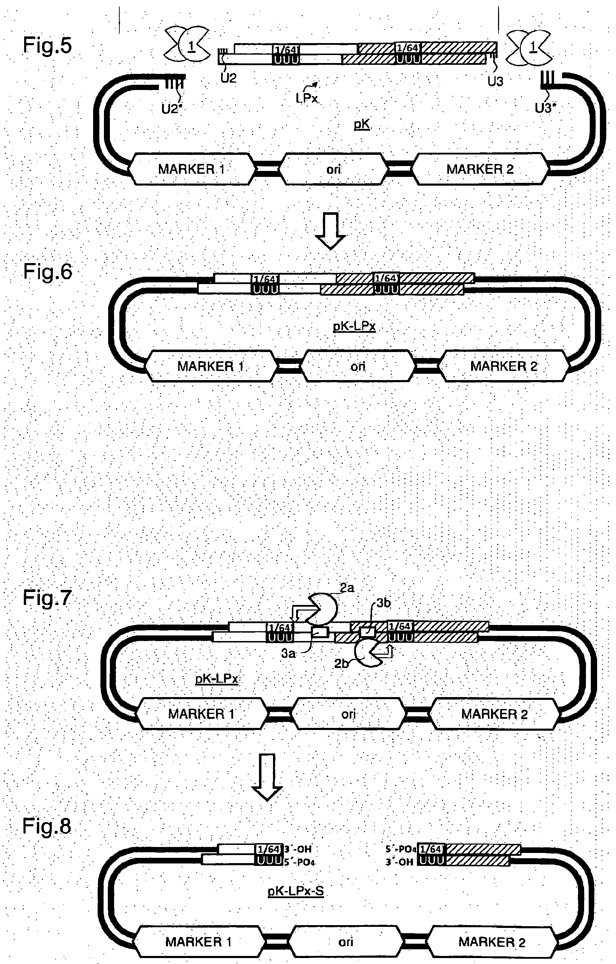

Das doppelsträngige DNA- Ligationsprodukt LPx verfügt über die definierten 3' Überhänge U2 und U3 des zweiten und der dritten Oligonukleotide O2, O3-x. Ein Klonierungsvektor pK wird vorbereitet, indem er beispielsweise mit zwei Restriktionsendonukleasen geschnitten und dephosphoryliert wird (vgl. Figur 5). Die Restriktionsendonukleasen sind so gewählt, das korrespondierende Überhänge U2* und U3* zu den definierten Überhängen U2, U3 der Ligationsprodukte LPx gebildet werden. Anschließend werden die Ligationsprodukte LPx mit Ligase 1 in den vorbereiteten Klonierungsvektor pK integriert, wodurch eine Bibliothek an Ligationsprodukt- Vektoren pK-LPx gebildet wird (Figur 6).

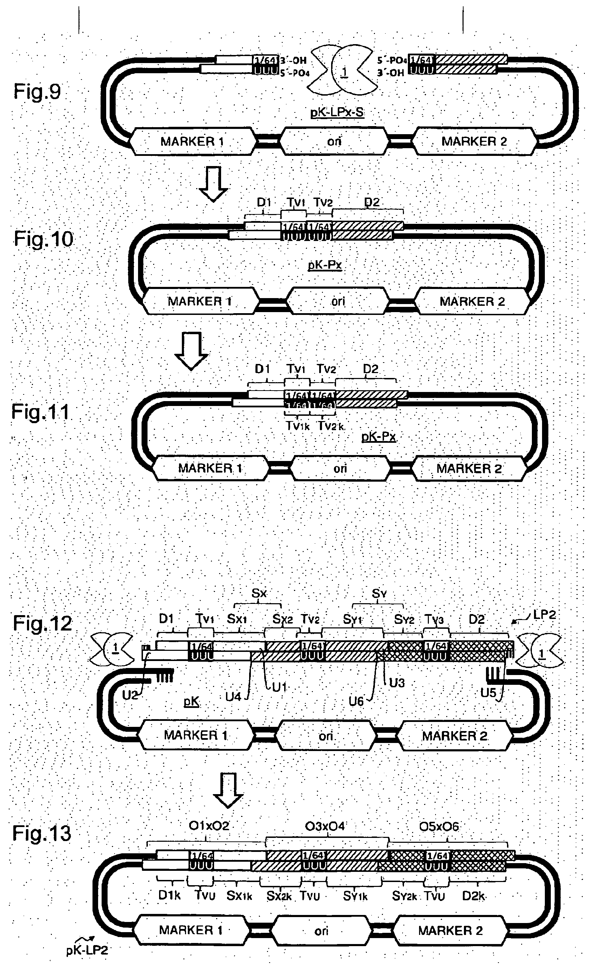

Die Spacer- Sequenz S der Ligationsprodukte LPx umfasst mindestens eine, vorzugsweise zwei Erkennungssequenzen 3a, 3b für mindestens eine Typ IIS Restriktionsendonuklease 2a, 2b (vgl. Figur 7). Typ IIS Restriktionsendonukleasen schneiden DNA "blunt", d.h. ohne Überhang, an einer Stelle in einem definierten Abstand zur Erkennungssequenz 3a, 3b. Die Erkennungssequenzen 3a, 3b für die Typ IIS Restriktionsendonukleasen 2a, 2b sind so innerhalb der Spacer- Sequenz S angeordnet, dass die erste Restriktionsendonuklease 2a direkt nach dem ersten variablen Codon-Triplett T_{V1} und die zweite Restriktionsendonuklease 2b direkt vor dem zweiten Codon-Triplett T_{V2} schneidet, so dass die Spacer- Sequenz S komplett aus dem Ligationsprodukt- Vektor pK-LPx herausgeschnitten wird. Dabei wird ein lineares Fragment pK-LPx-S gebildet, das aus Klonierungsvektor und Ligationsprodukt LPx minus Spacer- Sequenz S besteht (vgl. Figur 8). Das lineare Fragment pK-LPx-S wird mittels einer Ligase 1 zum Produktvektor pK-Px religiert (vgl. Figur 9 und 10). Der Produktvektor pK-Px umfasst nunmehr das gewünschte DNA- Fragment mit folgenden Teilbereichen: definierter erster Bereich D1 der Nukleinsäure- Bibliothek, erstes variables Codon- Triplett T_{V1}, zweites variables Codon- Triplett T_{V2} und definierter zweiter Bereich D2 der Nukleinsäure- Bibliothek.

Wie in Figur 11 dargestellt, werden in einem letzten Verfahrensschritt die Universal-Nukleotide durch "normale" Nukleotide A, T, C und G ersetzt. Die ist beispielsweise durch herkömmlich bekannte Klonierungsmethoden, so genannter nick replication, rolling circle, PCR o.ä. möglich. Eine weitere einfache Möglichkeit besteht darin, das Plasmid in einem geeigneten Wirt, beispielsweise in *E.coli* zu vervielfältigen. Dabei werden die Universal-Nukleotide der variablen Codon-Tripletts Tvu durch die Reparaturmaschinerie des Wirtes durch die komplementären "normalen" Nukleotide A, T, C und G ersetzt und bilden somit die komplementär variablen Codon- Tripletts T_{V1k} und T_{V2k}.

Figur 12 bis Figur 16 zeigen die Herstellung einer Nukleinsäure mit drei benachbarten variablen Codon- Tripletts. Die dabei verwendeten sechs Oligonukleotide sind tabellarisch aufgelistet:

| | |
|---|---|
| O1x | Oligonukleotid- Bibliothek: 5'- definierter erster Bereich D1 - erstes variables Codon- Triplett T_{V1} - erster Teilbereich Sx1 eines Spacers Sx - definierter 3' Überhang U1 |
| O2 | 5' - komplementäre Teilsequenz SX₁ₖ - Codon- Triplett T_{VU} - komplementäre Sequenz D1k - definierter 3' Überhang U2 |
| O3x | Oligonukleotid- Bibliothek: 5' - zweiter Teilbereich Sx₂ eines Spacers Sx - zweites variables Codon-Triplett T_{V2} - erster Teilbereich S_{Y1} eines Spacers S_{Y} - definierter 3' Überhang U3 |
| O4 | 5' - komplementäre Teilsequenz S_{Y1k} - Codon- Triplett T_{VU} - komplementäre Teilsequenz Sx₂ₖ - definierter 3' Überhang U4 |
| O5x | Oligonukleotid- Bibliothek: 5' - zweiter Teilbereich S_{Y2} eines Spacers S_{Y} - drittes variables Codon-Triplett T_{V3} - definierter zweiter Bereich D2 - definierter 3' Überhang U5 |
| O6 | 5' - komplementäre Sequenz D2k - Codon- Triplett Tvu - komplementäre Teilsequenz S_{Y2k} - definierter 3' Überhang U6 |

Für die definierten Uberhange gilt: U1 ist komplementär zu U4; U3 ist komplementär zu U6; U2 und U5 sind komplementär zu den durch Restriktion o.ä. hergestellten Überhängen eines Klonierungsvektors pK. Diese Überhänge können beispielsweise über unvollständige PCR, Ligation, independent cloning mit entsprechenden Polymerasen etc. hergestellt werden.

Die Oligonukleotide bzw. Oligonukleotid- Bibliotheken O1 **x** und O2, O3x und 04 sowie 05x und 06 werden jeweils miteinander hybridisiert. Anschließend werden die Oligonukleotid 1 - Oligonukleotid 2 - DNAs O1xO2 mit den Oligonukleotid 3 - Oligonukleotid 4- DNAs O3x04 und den Oligonukleotid 5 - Oligonukleotid 6- DNAs 05x06 mit einer Ligase 1 zur Bibliothek aus doppelsträngigem Ligationsprodukt LP2 verbunden (Figur 12). Die Ligationsprodukte LP2 umfassen jeweils folgende Teilbereiche: definierter erster Bereich D1 der Nukleinsäure- Bibliothek, erstes variables Codon- Triplett T_{V1}, erste Spacer- Sequenz Sx, zweites variables Codon- Triplett T_{V2}, zweite Spacer-Sequenz S_{Y}, drittes variables Codon- Triplett T_{V3} und definierter zweiter Bereich D2 der Nukleinsäure- Bibliothek.

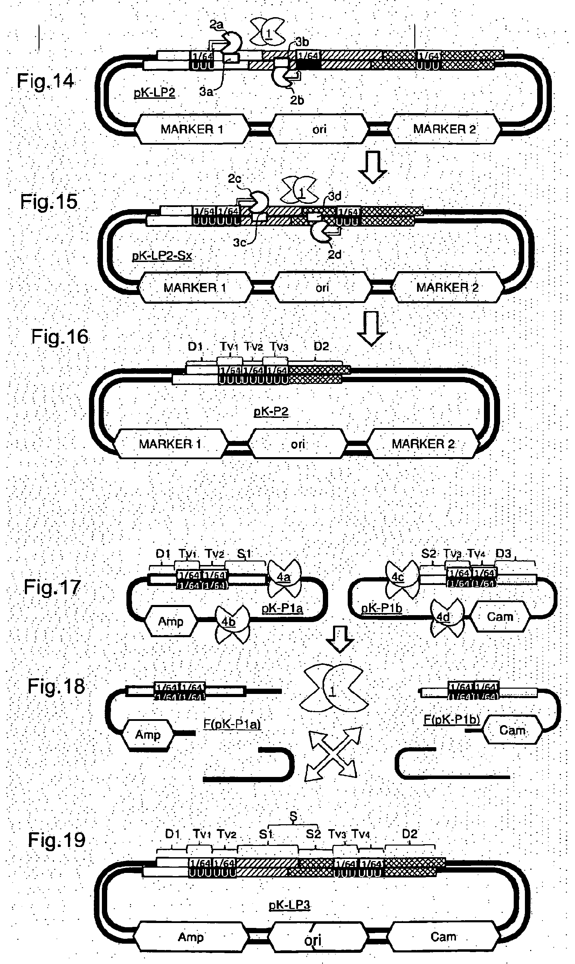

Die Ligationsprodukte LP2 werden über die Überhänge U2 und U5 in einen entsprechend vorbereiteten Klonierungsvektor pK integriert, wodurch Ligationsprodukt-Vektoren pK-LP2 gebildet werden (Figur 13). Die Spacer- Sequenzen Sx und S_{Y} der Ligationsprodukte LP2 umfassen jeweils mindestens eine, vorzugsweise zwei Erkennungssequenzen 3a - 3d für mindestens eine Typ IIS Restriktionsendonuklease 2a - 2d (vgl. Figur 14 und Figur 15). Die Erkennungssequenzen 3a- 3d für die Typ IIS Restriktionsendonukleasen 2a - 2d sind so innerhalb der Spacer- Sequenzen Sx und S_{Y} angeordnet, dass die eine Restriktionsendonuklease 2a, 2c direkt nach dem ersten variablen Codon- Triplett T_{V1} bzw. direkt nach dem zweiten variablen Codon- Triplett T_{V2} und die andere Restriktionsendonuklease 2b, 2d direkt vor dem zweiten bzw. dritten Codon- Triplett T_{V2}, T_{V3} schneidet, so dass die Spacer- Sequenzen Sx und S_{Y} in zwei auf einander folgenden Verfahrensschritten aus den Ligationsprodukt- Vektoren pK-LP2 herausgeschnitten werden können. Wichtig ist hierbei jedoch, dass die Spacer-Sequenzen Sx und S_{Y} jeweils unterschiedliche Erkennungssequenzen 3a bis 3d für die Restriktionsendonukleasen 2a bis 2d aufweisen, damit gezielt zuerst eine Spacer-Sequenz Sx oder S_{Y} und anschließend die zweite Spacer- Sequenz S_{Y} oder Sx entfernt werden kann.

Figur 14 zeigt, dass zuerst die Spacer- Sequenz Sx mit Hilfe der Typ IIS Restriktionsendonukleasen 2a und 2b aus den Ligationsprodukt- Vektoren pK-LP2 herausgeschnitten werden. Der geschnittene Vektor wird zum Vektor pK-LP2-Sx (vgl. Figur 15) religiert. Die zweite Spacer- Sequenz S_{Y} wird nunmehr aus dem Vektor pK-LP2-Sx mit Hilfe von zwei anderen Typ IIS Restriktionsendonukleasen 2c und 2d herausgeschnitten. Nach Religation mit Ligase 1 erhält man den Produktvektor pK-P2 mit dem gewünschten DNA Fragment. Dieses umfasst insbesondere drei aufeinanderfolgende variable Codon- Tripletts. Das im Produktvektor pK-P2 enthaltene DNA- Fragment weist folgende Teilbereiche auf: definierter erster Bereich D1 der Nukleinsäure- Bibliothek, erstes variables Codon- Triplett T_{V1}, zweites variables Codon-Triplett T_{V2}, drittes variables Codon- Triplett T_{V3} und definierter zweiter Bereich D2 der Nukleinsäure- Bibliothek (Figur 16). Analog zu Figur 11, werden in einem letzten Verfahrensschritt die Universal- Nukleotide im Produktvektor pK-P2 durch die "normalen" Nukleotide ersetzt (nicht dargestellt).

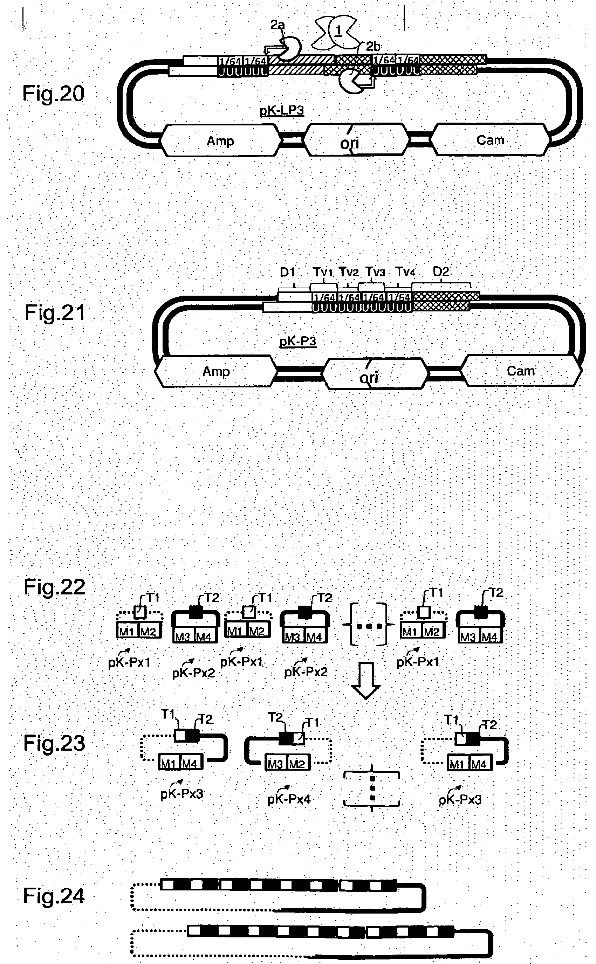

Figuren 17 bis 21 zeigen eine Möglichkeit, zwei erfindungsgemäß hergestellte Bibliotheken aus DNA- Fragmenten miteinander zu einer Bibliothek mit einem größeren DNA- Fragment mit folgender Sequenz zu verbinden: definierter erster Bereich D1 der Nukleinsäure- Bibliothek, erstes variables Codon- Triplett T_{V1}, zweites variables Codon-Triplett T_{V2}, drittes variables Codon- Triplett T_{V3}, viertes variables Codon- Triplett T_{V4} und definierter zweiter Bereich D2 der Nukleinsäure- Bibliothek (Figur 21). Zuerst werden zwei Bibliotheken pk-P1 a und pK-P1 b mit jeweils zwei benachbarten variablen Codon- Tripletts gemäß den in den Figuren 1 bis 11 beschriebenen Verfahrensschritten hergestellt, wobei pk-P1a einen definierten erster Bereich D1 der Nukleinsäure- Bibliothek, ein erstes variables Codon- Triplett T_{V1}, ein zweites variables Codon- Triplett T_{V2}, und eine erste Teilsequenz S1 einer Spacer- Sequenz S umfasst und wobei pK-P1b eine zweite Teilsequenz S2 einer Spacer- Sequenz S, ein drittes variables Codon- Triplett T_{V3}, ein viertes variables Codon- Triplett T_{V4} und einen definierten zweiten Bereich D2 der Nukleinsäure- Bibliothek umfasst.

Die Ausgangsplasmide der ersten Bibliothek pK-P1 a umfassen jeweils mindestens ein erstes Resistenzgen MARKER 1, beispielsweise eine Ampicillin (Amp)- Resistenz. Die Ausgangsplasmide der zweiten Bibliothek pK-P1b umfassen jeweils mindestens ein zweites Resistenzgen MARKER 2, beispielsweise eine Cam- Resistenz. Die Vektoren der ersten Bibliothek pK-P1 a werden mit zwei Restriktionsendonuklease 4a und 4b geschnitten, wobei die erste Restriktionsendonuklease 4a vorzugsweise blunt nach dem letzten Nukleotid der ersten Teilsequenz S1 der Spacer- Sequenz S schneidet. Zudem werden die Vektoren der ersten Bibliothek pK-P1 a mit einer zweiten Restriktionsendonuklease 4b im Origin of Replication geschnitten. Dabei entsteht die Fragment- Bibliothek F(pK-P1a), mit jeweils dem o.g. ersten DNA- Fragment und dem ersten Resistenz- Markergen Amp. Die Vektoren der zweiten Bibliothek pK-P1 b werden mit einer dritten Restriktionsendonuklease 4c vorzugsweise blunt vor dem ersten Nukleotid der zweiten Teilsequenz S2 der Spacer- Sequenz S geschnitten. Zudem werden die Vektoren der zweiten Bibliothek pK-P1 b wird mit einer vierten Restriktionsendonuklease 4d im Origin of Replication geschnitten. Dabei entsteht die Fragment- Bibliothek F(pK-P1b), mit dem o.g. zweiten DNA- Fragment und dem zweiten Resistenz- Markergen Cam. Als Restriktionsnukleasen 4b, 4d, die die Vektoren pK-P1a und pK-P1 b jeweils im Origin of Replication schneiden, werden vorzugsweise dieselben Restriktionsendonukleasen verwendet, d.h. Restriktionsendonuklease 4b = Restriktionsendonuklease 4d.

Die beiden Fragment- Bibliotheken F(pK-P1a) und F(pK-P1b) werden nunmehr mittels Ligase 1 miteinander verbunden (Figur 18). Dabei entsteht eine Bibliothek aus Ligationsprodukt- Vektor pK-LP3 (Figur 19). Dieser umfasst ein DNA- Sequenz, bestehend aus einem definierten ersten Bereich D1 der Nukleinsäure- Bibliothek, erstes variables Codon- Triplett T_{V1}, zweites variables Codon- Triplett T_{V2}, Spacer- Sequenz S, drittes variables Codon- Triplett T_{V3}, viertes variables Codon- Triplett T_{V4} und definierter zweiter Bereich D2 der Nukleinsäure- Bibliothek sowie die beiden Resistenz- Markergene Amp und Cam. Über die Ligation werden die durch die Restriktion mit den Restriktionsendonukleasen 4b, 4d entstandenen Teilfragmente der geschnittenen Origin of Replication wiederum zu einem funktionalen Origin of Replication zusammengefügt. Die Ligationsprodukt- Vektoren pK-LP3 können über Doppelselektion auf Amp und Cam gezielt selektioniert werden. Da die Ligationsprodukt- Vektoren pK-LP3 einen funktionalen Origin of Replication umfassen, können diese einfach in einem geeigneten Organismus, beispielsweise in *E.coli,* repliziert werden.

Die Spacer- Sequenz S wird anschließend über Typ IIS Restriktionsendonuklease 2a, 2b und Religation mit Ligase 1 aus den Ligationsprodukt- Vektoren pK-LP3 entfernt (Figur 20), wodurch die Produktvektoren pK-P3 mit der gewünschten DNA- Sequenz gebildet werden (Figur 21).

Alternativ können auch in einem Verfahren gemäß den Figuren 1 bis 11 vier Oligonukleotide 05b, 06, 07b und 08 (siehe Tabelle) verwendet werden, die jeweils direkt zwei benachbarte variable Codon- Tripletts umfassen, d.h.

| | |
|---|---|
| O5b | 5'- definierter erster Bereich D1 - erstes variables Codon- Triplett T_{V1} - zweites variables Codon- Triplett T_{V2} - erster Teilbereich S1 eines Spacers S - definierter 3' Überhang U5 |
| O6 | 5' - komplementäre Teilsequenz S1k - Codon- Triplett T_{VU} - Codon- Triplett T_{VU} - komplementäre Sequenz D1k - definierter 3' Überhang U6 |
| O7b | 5' - zweiter Teilbereich S2 eines Spacers Sx - drittes variables Codon- Triplett T_{V3} - viertes variables Codon- Triplett T_{V4}- definierter zweiter Bereich D2 - definierter 3' Überhang U7 |
| O8 | 5' - komplementäre Sequenz D2k - Codon- Triplett T_{VU} - Codon- Triplett T_{VU} - komplementäre Teilsequenz S2k - definierter 3' Überhang U8 |

Dabei entsteht direkt eine Produkt- Bibliothek mit jeweils der gewünschten DNA-Sequenz entsprechend Figur 21.

Figur 22 bis 24 zeigen schematisch das Erstellen von Nukleinsäure- Bibliotheken, bei denen eine Mehrzahl von variablen Codon- Tripletts nebeneinander angeordnet sind. Dies erfolgt durch Kombinationen mehrerer Bibliotheken aus entsprechend konstruierten Produktvektoren pK-Px1, pK-Px2, die jeweils Sequenzen für weitere Spacer- Elemente anstelle des definierten ersten oder zweiten Bereichs der Nukleinsäure- Bibliothek enthalten. Insbesondere ist hierfür auch die Verwendung mehrerer unterschiedlicher Markergene M1 - M4 notwendig bzw. sinnvoll.

Durch Kombinationen mehrerer entsprechend konstruierter Bibliotheken von ersten, zweiten usw. Produktvektoren pK-Px1, pK-Px2 mit Sequenzen für weitere Spacer Elemente anstelle des definierten ersten oder zweiten Bereichs der NukleinsäureBibliothek, können somit Nukleinsäure- Sequenzen mit eine beliebigen Anzahl nebeneinander angeordneter variabler Codon- Tripletts erzeugt werden. Gemäß einer Ausführungsform umfasst eine erste Bibliothek von Produktvektor pK-Px1 mit einem ersten variablen Nukleinsäure- Bereich mindestens zwei unterschiedliche Selektionsmarker M1 und M2. Eine zweite Bibliothek von Produktvektor pK-Px2 mit einem zweiten variablen Nukleinsäure- Bereich umfasst mindestens zwei weitere unterschiedliche Selektionsmarker M3 und M4. Die zwei Produkt- Bibliotheken pK-Px1, pK-Px2 werden jeweils an zwei Stellen mittels geeigneter Restriktionsendonukleasen geschnitten und die dabei entstandenen Fragmente werden miteinander durch Religation neu kombiniert (vgl. auch Figuren 17 bis 21). Dabei entstehen Bibliotheken von ProduktVektoren (vgl. Figur 23), die beispielsweise zweimal den ersten variablen Nukleinsäure-Bereich, zweimal den zweiten variablen Nukleinsäure- Bereich, die Kombination aus Nukleinsäure- Bereich eins und Nukleinsäure- Bereich zwei (= Bibliothek pK-Px3) oder die Kombination aus Nukleinsäure- Bereich zwei und Nukleinsäure- Bereich eins (= Bibliothek pK-Px4) aufweisen. Die Bibliotheken mit der gewünschten Nukleinsäure- Kombination können durch gezielte Selektion anhand der Selektionsmarker M1 - M4 identifiziert werden. Beispielsweise kann die Produkt- Bibliothek pK-Px1 durch Doppelselektion auf Marker M1 und Marker M2 und Produkt- Bibliothek pK-Px2 durch Doppelselektion auf Marker M3 und Marker M4 selektiert werden.

Durch weitere Kombination können somit Nukleinsäure- Bibliotheken mit einer Vielzahl von benachbarten variablen Codon- Tripletts konstruiert werden (Figur 24).

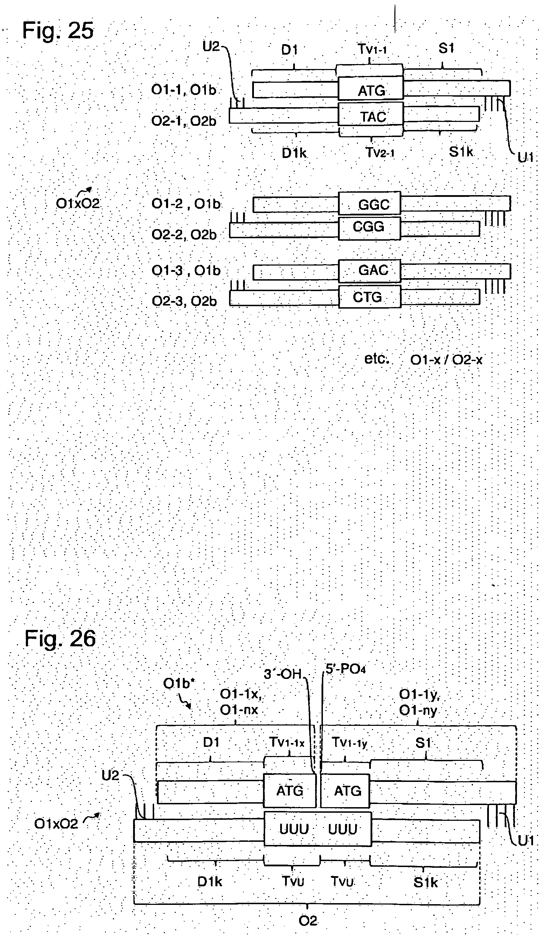

Figur 25 zeigt eine weitere Ausführungsform von beim erfindungsgemäßen Verfahren verwendeten Oligonukleotiden. Hierbei werden die oben beschriebenen Oligonukleotide O1-1 bis O1-x einer ersten Oligonukleotid- Bibliothek O1b jeweils mit den entsprechenden weitgehend komplementären Oligonukleotiden O2-1 bis O2-x einer ersten Komplementär- Oligonukleotid- Bibliothek O2b hybridisiert, wobei die Oligonukleotide O2-1 bis O2-x der ersten Komplementär- Oligonukleotid- Bibliothek O2b jeweils folgende Teilsequenzen aufweisen: erste Teilsequenz S1 k, die komplementär zum ersten Teilbereich S1 des Spacer- Elementes S (vgl. Figur 1) der ersten Oligonukleotid-Bibliothek O1b ausgebildet ist, komplementäres Codon- Triplett T_{V2-1} bis T_{V2-x} zu einem der Oligonukleotide O1-1 bis O1-x aus der ersten Oligonukleotid- Bibliothek O1b und eine Teilsequenz D1k, die komplementär zum definierten ersten Bereich D1 der Oligonukleotide O1-1 bis O1-x der ersten Oligonukleotid- Bibliothek O1b aufgebaut ist. Zu jedem Oligonukleotid O1-1 bis O1-x der ersten Oligonukleotid- BibliothekO1b gibt es in der ersten Komplementär- Oligonukleotid- Bibliothek O2b ein weitgehend komplementäres Oligonukleotid O2-1 bis O2-x. Die Oligonukleotide O1-x und O2-x werden jeweils nur als weitgehend komplementär bezeichnet, da zumindest die Oligonukleotide O1-x der ersten Oligonukleotid- Bibliothek O1b einen 3' Überhang U1 gegenüber den jeweils komplementären Oligonukleotiden O2-x der ersten Komplementär-Oligonukleotid- Bibliothek O2b aufweisen. Weiterhin weisen die Oligonukleotide O2-x der ersten Komplementär- Oligonukleotid- Bibliothek O2b vorzugsweise einen 3' Überhang gegenüber den jeweils komplementären Oligonukleotiden O1-x der ersten Oligonukleotid-Bibliothek O1b auf.

Bei dieser Ausführungsform werden also jeweils zwei, bis auf die 3' Überhänge komplementäre Oligonukleotide O1-x und O2-x miteinander hybridisiert. Dies ist insbesondere vorteilhaft, wenn nur wenige unterschiedliche Codon- Tripletts getestet werden sollen. Die Vielzahl der jeweils hybridisierten Oligonukleotide O1-x / O2-x bilden zusammen die erste doppelsträngige DNA- Bibliothek O1xO2. Anstelle des so genannten zweiten komplementären Oligonukleotids 04 (vgl. Figuren 1 bis 11) kann analog eine zweite Komplementär- Oligonukleotid- Bibliothek 04b (nicht dargestellt) verwendet werden.

Figur 26 zeigt ebenfalls eine weitere Ausführungsform der beim erfindungsgemäßen Verfahren verwendeten Oligonukleotide, wobei die Ausgangs- Oligonukleotide bereits zwei benachbarte variable Codons- Tripletts umfassen. Hierbei ist vorgesehen, dass die erste Oligonukleotid- Bibliothek O1b* aus jeweils zwei Oligonukleotiden O1-1x bis O1-nx und O1-1y bis O1-ny bestehen, wobei das erste Oligonukleotid O1-1x folgende Teilsequenzen aufweist: 5'- definierter erster Bereich D1 - erstes variables Codon- Triplett T_{V1-1x} und wobei das zweite Oligonukleotid O1-1y folgende Teilsequenzen aufweist: zweites variables Codon- Triplett T_{V1-1y} - erster Teilbereich S1 eines Spacers S und definierter 3' Überhang U1. Das erste komplementäre Oligonukleotid O2 weist dementsprechend folgende Teilsequenzen auf: 5'-komplementäre Teilsequenz S1k-Codon- Triplett Tvu - Codon- Triplett Tvu - komplementäre Sequenz D1k - definierter 3' Überhang U2. Nach Hybridisierung und Ligation der drei Oligonukleotide O1-nx, O1-ny und O2 entstehen DNA- Ligationsprodukte mit der Sequenz: definierter erster Bereich D1 - erstes variables Codon- Triplett T_{V1-1x} - zweites variables Codon- Triplett T_{V1-1y} - erster Teilbereich S1 eines Spacers S. Diese werden beispielsweise bei einem Verfahren gemäß Figuren 17 bis 19 eingesetzt.

Die Erfindung wurde unter Bezugnahme auf eine bevorzugte Ausführungsform beschrieben. Es ist jedoch für einen Fachmann vorstellbar, dass Abwandlungen oder Änderungen der Erfindung gemacht werden können, ohne dabei den Schutzbereich der nachstehenden Ansprüche zu verlassen. Insbesondere soll durch die Erfindung auch die Verwendung abgewandelter Oligonukleotide umfasst sein, die nach Hybridisierung und Ligation entsprechende doppelsträngige DNA- Moleküle bilden.

### Bezugszeichenliste

1 Ligase
2 Typ IIS Restriktionsendonuklease
3 Erkennungssequenz
4 Restriktionsendonuklease
D definierter Bereich
A Nukleotid Adenin
Amp Ampicillin Resistenzgen
C Nukleotid Cytosin
Cam Cam (Chloramphenicol) Resistenzgen
F Fragment
G Nukleotid Guanin
LP Ligationsprodukt
ori Origin of Replication
O Oligonukleotid
O b Oligonukleotid- Bibliothek
O1xO2 Ligationsprodukt- Bibliothek
P Produkt
pK Klonierungsvektor
pK-LP Ligationsprodukt- Vektor
pK-P Produkt- Vektor
S Spacer- Sequenz
T Nukleotid Thymin
Tv variables Codon- Triplett
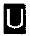 Universal Nukleotid
U1 - U4 3' Überhang
U2*, U3* 3' Überhang

## Patentansprüche

1. Verfahren zur Herstellung einer DNA- Sequenz für eine Nukleinsäure- Bibliothek mit einer Vielzahl von Elementen,
wobei die DNA- Sequenz jeweils mindestens drei Bereiche umfasst,
wobei der ersten und der dritte Bereich in jedem Element jeweils definierte Sequenzen (D1, D2) aufweist, die in allen Elementen der Nukleinsäure- Bibliothek jeweils identisch sind,
wobei ein mittlerer zweiter Bereich jeweils mindestens zwei benachbarte variable Codon- Tripletts umfasst,
wobei bei dem Verfahren mindestens zwei Oligonukleotid- Bibliotheken und mindestens zwei einzelsträngige Oligonukleotide mit den nachfolgenden Eigenschaften verwendet werden:
• eine erste Oligonukleotid- Bibliothek (O1b) bestehend aus Oligonukleotiden (O1-x) mit jeweils einer Teilsequenz, die dem definierten ersten Bereich (D1) der Nukleinsäure- Bibliothek entspricht, mindestens einem ersten variablen Codon-Triplet (T_{V1}), einer daran anschließenden ersten Teilsequenz (S1) eines Spacers (S) und einem definierten 3' Überhang (U1),
• ein erstes komplementäres Oligonukleotid (O2) oder eine erste Komplementär-Oligonukleotid- Bibliothek, wobei das erste komplementäre Oligonukleotid (O2) weitgehend komplementär zu den Oligonukleotiden (O1-x) der ersten Oligonukleotid- Bibliothek (O1b) ist, mit einer Teilsequenz (S1 k), die komplementär zur ersten Teilsequenz (S1) des Spacers (S) der Oligonukleotide (O1-x) der ersten Oligonukleotid- Bibliothek (O1b) ausgebildet ist, mindestens einem ersten komplementär- variablen bzw. weitgehend komplementär- variablen Codon- Triplet (Tvu) und einer Teilsequenz (D1 k), die komplementär zum ersten Bereich (D1) der Oligonukleotide (O1-x) der ersten Oligonukleotid- Bibliothek (O1b) ausgebildet ist,
• eine zweite Oligonukleotid- Bibliothek (O3b) bestehend aus Oligonukleotiden (O3-x) mit einer Teilsequenz, die die zweite Teilsequenz (S2) des Spacers (S) bildet, mindestens einem zweiten variablen Codon- Triplet (T_{V2}) und einer Teilsequenz, die dem definierten zweiten Bereich (D2) der Nukleinsäure- Bibliothek entspricht; und
• ein zweites komplementäres Oligonukleotid (04) oder eine zweite Komplementär-Oligonukleotid- Bibliothek, wobei das zweite komplementäre Oligonukleotid (04) weitgehend komplementär zu den Oligonukleotiden (O3-x) der zweiten Oligonukleotid- Bibliothek (O3b) ist, mit einer Teilsequenz (D2k), die komplementär zum zweiten Bereich (D2) der zweiten Oligonukleotid- Bibliothek (O3b) ausgebildet ist, mindestens einem zweiten komplementär- variablen bzw. weitgehend komplementär- variablen Codon- Triplet (T₂ₖ), mit einer Teilsequenz (S2k), die komplementär zur zweiten Teilsequenz (S2) des Spacers (S) der Oligonukleotide (O3-x) der zweiten Oligonukleotid- Bibliothek (O3b) ausgebildet ist und einen daran anschließenden definierten 3' Überhang (U4);
ODER
• eine erste Oligonukleotid- Bibliothek (O1b) bestehend aus Oligonukleotiden (O1-x) mit jeweils einer Teilsequenz, die dem definierten ersten Bereich (D1) der Nukleinsäure- Bibliothek entspricht, mindestens einem ersten variablen Codon-Triplet (T_{V1}) und einer daran anschließenden ersten Teilsequenz (S1) eines Spacers (S),
• ein erstes komplementäres Oligonukleotid (O2) oder eine erste Komplementär-Oligonukleotid- Bibliothek, wobei das erste komplementäre Oligonukleotid (O2) weitgehend komplementär zu den Oligonukleotiden (O1-x) der ersten Oligonukleotid- Bibliothek (O1b) ist, mit einem definierten 5' Überhang, mit einer Teilsequenz (S1k), die komplementär zur ersten Teilsequenz (S1) des Spacers (S) der Oligonukleotide (O1-x) der ersten Oligonukleotid- Bibliothek (O1b) ausgebildet ist, mindestens einem ersten komplementär- variablen bzw. weitgehend komplementär- variablen Codon- Triplet (Tvu) und einer Teilsequenz (D1k), die komplementär zum ersten Bereich (D1) der Oligonukleotide (O1-x) der ersten Oligonukleotid- Bibliothek (O1b) ausgebildet ist,
• eine zweite Oligonukleotid- Bibliothek (O3b) bestehend aus Oligonukleotiden (O3-x) mit einem definierten 5' Überhang, mit einer Teilsequenz, die die zweite Teilsequenz (S2) des Spacers (S) bildet, mindestens einem zweiten variablen Codon- Triplet (T_{V2}) und einer Teilsequenz, die dem definierten zweiten Bereich (D2) der Nukleinsäure- Bibliothek entspricht;
• ein zweites komplementäres Oligonukleotid (04) oder eine zweite Komplementär-Oligonukleotid- Bibliothek, wobei das zweite komplementäre Oligonukleotid (04) weitgehend komplementär zu den Oligonukleotiden (O3-x) der zweiten Oligonukleotid- Bibliothek (O3b) ist, mit einer Teilsequenz (D2k), die komplementär zum zweiten Bereich (D2) der zweiten Oligonukleotid- Bibliothek (O3b) ausgebildet ist, mindestens einem zweiten komplementär- variablen bzw. weitgehend komplementär- variablen Codon- Triplet (T₂ₖ) und mit einer Teilsequenz (S2k), die komplementär zur zweiten Teilsequenz (S2) des Spacers (S) der Oligonukleotide (O3-x) der zweiten Oligonukleotid- Bibliothek (O3b) ausgebildet ist;
wobei folgende Verfahrensschritte durchgeführt werden:
• Hybridisierung der Oligonukleotide (O1-x) der ersten Oligonukleotid- Bibliothek (O1b) mit dem ersten weitgehend komplementären Oligonukleotid (O2) zu einer ersten doppelsträngigen DNA- Bibliothek (O1xO2) sowie Hybridisierung der Oligonukleotide (O3-x) der zweiten Oligonukleotid- Bibliothek (O3b) mit dem zweiten weitgehend komplementären Oligonukleotid (04) zu einer zweiten doppelsträngigen DNA- Bibliothek (O3x04);
• Ligation der ersten doppelsträngigen DNA- Bibliothek (O1 xO2) mit der zweiten doppelsträngigen DNA- Bibliothek (O3x04) unter Ausbildung einer Bibliothek mit doppelsträngigen DNA- Ligationsprodukten (LPx), die folgende Teilbereiche umfassen: definierter ersten Bereich (D1) der Nukleinsäure- Bibliothek, mindestens ein erstes variables Codon- Triplett (T_{V1}), Spacer- Sequenz (S), mindestens ein zweites variables Codon- Triplett (T_{V2}) und definierter zweiter Bereich (D2) der Nukleinsäure- Bibliothek;
• Entfernen der Spacer- Sequenz (S) aus der Bibliothek von doppelsträngigem DNA- Ligationsprodukt (LPx) unter Ausbildung einer Bibliothek von doppelsträngigem DNA- Produkt (Px), das folgende Teilbereiche umfasst: definierter ersten Bereich (D1) der Nukleinsäure- Bibliothek, mindestens ein erstes variables Codon- Triplet (T_{V1}), mindestens ein zweites variables Codon- Triplet (T_{V2}) und definierter zweiter Bereich (D2) der Nukleinsäure- Bibliothek.

2. Verfahren nach Anspruch 1, wobei das mindestens eine erste komplementärvariable Codon- Triplet (Tvu) und das mindestens eine zweite komplementärvariable Codon- Triplet (Tvu) jeweils drei "Universal- Basen" (U) oder ein Gemisch aus allen vier kanonischen Nukleotiden (A, T, C, G) umfassen.

3. Verfahren nach Anspruch 1 oder 2, wobei die definierten 3' Überhänge (U1, U4) der Oligonukleotide (O1-x) der ersten Oligonukleotid- Bibliothek (O1b) und des zweiten komplementären Oligonukleotids (04) dieselbe Anzahl von Nukleotiden umfassen und zueinander zumindest weitgehend komplementär sind oder wobei die definierten 5' Überhänge der Oligonukleotide (O2-x) der zweiten Oligonukleotid-Bibliothek (O2b) und des ersten komplementären Oligonukleotids (O3) dieselbe Anzahl von Nukleotiden umfassen und zueinander zumindest weitgehend komplementär sind.

4. Verfahren nach einem der voranstehenden Ansprüche, wobei die Oligonukleotide (O1-x) der ersten Oligonukleotid- Bibliothek (O1b) und das zweite komplementäre Oligonukleotid (04) jeweils definierte, zueinander zumindest weitgehend komplementäre 3' Überhänge (U1, U4) aufweisen und wobei das erste komplementäre Oligonukleotid (O2) und die Oligonukleotide (O3-x) der zweiten Oligonukleotid- Bibliothek (O3b) jeweils definierte 3' Überhänge (U2, U3) aufweisen, die jeweils unterschiedlich lang und nicht komplementär zueinander ausgebildet sind und wobei die definierten 3' Überhänge (U) des ersten komplementären Oligonukleotids (O2) und der Oligonukleotide (O3-x) der zweiten Oligonukleotid-Bibliothek (O3b) jeweils unterschiedlich lang und nicht komplementär zu den definierten 3' Überhänge (U1, U4) der Oligonukleotide (O1-x) der ersten Oligonukleotid- Bibliothek (O1b) und des zweiten komplementären Oligonukleotids (04) ausgebildet sind.

5. Verfahren nach einem der Ansprüche 1 bis 3, wobei die Oligonukleotide (O3-x) der zweiten Oligonukleotid- Bibliothek (O3b) und das erste komplementäre Oligonukleotid (O2) jeweils definierte, zueinander zumindest weitgehend komplementäre 5' Überhänge aufweisen und wobei das zweite komplementäre Oligonukleotid (04) und die Oligonukleotide (O1-x) der ersten Oligonukleotid- Bibliothek (O1b) jeweils definierte 5' Überhänge aufweisen, die jeweils unterschiedlich lang und nicht komplementär zueinander ausgebildet sind und wobei die definierten 5' Überhänge des zweiten komplementären Oligonukleotids (04) und der Oligonukleotide (O1-x) der ersten Oligonukleotid- Bibliothek (O1b) jeweils unterschiedlich lang und nicht komplementär zu den definierten 5' Überhänge der Oligonukleotide (O3-x) der zweiten Oligonukleotid- Bibliothek (O3b) und des ersten komplementären Oligonukleotids (O2) ausgebildet sind.

6. Verfahren nach einem der voranstehenden Ansprüche, wobei die Spacer-Sequenz (S) mindestens eine Erkennungssequenz (3) für mindestens eine Typ IIS Restriktionsendonuklease (2) umfasst, wobei die mindestens eine Typ IIS Restriktionsendonuklease (2) das doppelsträngige DNA - Ligationsprodukt (LPx) 3' nach dem dritten Nukleotid des ersten variablen Codon- Triplets (T_{V1}) und / oder 5' vor dem ersten Nukleotid des zweiten variablen Codon- Triplets (T_{V2}) schneidet.

7. Verfahren nach einem der voranstehenden Ansprüche, wobei die erste Oligonukleotid- Bibliothek (O1b) zwei benachbarte oder weitgehend benachbarte variable Codon- Triplets (T_{V1}, T_{V2}) und / oder die zweite Oligonukleotid- Bibliothek (O3b) zwei benachbarte oder weitgehend benachbarte variable Codon- Triplets (T_{V3}, T_{V4}) und wobei das erste und / oder das zweite Oligonukleotid (O2, 04) jeweils zwei benachbarte oder weitgehend benachbarte komplementär- variable Codon- Triplets (Tvu) aus jeweils drei "Universal- Basen" (U) oder ein Gemisch aus allen vier kanonischen Nukleotiden (A, T, C, G) umfassen.

8. Verfahren nach Anspruch 7, wobei durch die in Anspruch 1 beschriebenen Verfahrensschritte aus den zwei Oligonukleotid- Bibliotheken (O1b, O3b) und den zwei Oligonukleotiden (O2, 04) gemäß Anspruch 7 eine Nukleinsäure- Sequenz mit drei oder vier benachbarten variablen Codon- Triplets (T_{V1} - T_{V4}) hergestellt wird.

9. Verfahren nach Anspruch 6, wobei
• die Bibliothek von doppelsträngigem DNA- Ligationsprodukt (LPx) über die definierten 3' Überhänge (U2, U3) des ersten Oligonukleotids (O2) und des jeweiligen Oligonukleotids (O3-x) der zweiten Oligonukleotid- Bibliothek (O3b) durch Ligation in einen entsprechend vorbereiteten Klonierungsvektor (pK) mit korrespondieren Überhängen (U2*; U3*) integriert und eine Bibliothek von Ligationsprodukt- Vektor (pK-LPx) gebildet wird;
• die Bibliothek von Ligationsprodukt- Vektor (pK-LPx) mit mindestens einer Typ IIS Restriktionsendonuklease (2a, 2b) behandelt wird, wobei die Spacer- Sequenz (S) herausgeschnitten wird;
• die Bibliothek von geschnittenem Ligationsprodukt- Vektor (pK-LPx-S) religiert und zur Bibliothek von Produktvektor (pK-Px) cyclisiert wird;
• die Bibliothek von Produktvektor (pK-Px) ein doppelsträngiges DNA- Produkt (Px) mit folgenden Teilbereichen umfasst: definierter ersten Bereich (D1) der Nukleinsäure- Bibliothek, erstes variables Codon- Triplet (T_{V1}), zweites variables Codon- Triplet (T_{V2}) und definierter zweiter Bereich (D2) der NukleinsäureBibliothek.

10. Verfahren nach Anspruch 9, wobei eine erste Bibliothek von Produktvektor (pK-Px1) mit einer ersten variablen Nukleinsäuresequenz (T1) mindestens zwei unterschiedliche Selektionsmarker (M1, M2) umfasst, wobei eine zweite Bibliothek von Produktvektor (pK-Px2) mit einer zweiten variablen Nukleinsäuresequenz (T2) mindestens zwei weitere unterschiedliche Selektionsmarker (M3, M4) umfasst, wobei die beiden Bibliotheken von Produktvektor (pK-Px1, pK-Px2) jeweils an zwei Stellen mittels geeigneter Restriktionsendonukleasen geschnitten und die Fragmente miteinander durch Religation kombiniert werden, wobei die gewünschten Produktvektoren (pK-Px, pK-Px4) anhand der neu entstandenen Selektionsmarker-Kombination (M1/M4 bzw. M3/ M2) gezielt selektioniert werden.

## Claims

1. Method for producing a DNA sequence for a nucleic acid library comprising a plurality of elements,
wherein the DNA sequence comprises at least three sections,
wherein the first and the third section in each element have defined sequences (D1, D2) that are identical in each element of the nucleic acid library,
wherein a central second section comprises at least two adjoining variable codon triplets,
wherein at least two oligonucleotide libraries and at least two single stranded oligonucleotides with the following characteristics are used in this method:
• a first oligonucleotide library (O1b) consisting of oligonucleotides (O1-x) each comprising a partial sequence corresponding to the defined first section (D1) of the nucleic acid library, at least one first variable codon triplet (T_{V1}), a subsequently arranged first partial sequence (S1) of a spacer element (S) and a defined 3' overhang (U1);
• a first complementary oligonucleotide (O2) or a first complementary oligonucleotide library, wherein the first complementary oligonucleotide (O2) is substantially complementary to the oligonucleotides (O1-x) of the first oligonucleotide library (O1b), comprising a partial sequence (S1k) that is complementary to the first partial sequence (S1) of the spacer element (S) of the oligonucleotides (O1-x) of the first oligonucleotide library (O1b), at least one first complementary variable or substantially complementary variable codon triplet (Tvu) and a partial sequence (D1k) complementary to the first section (D1) of the oligonucleotides (O1-x) of the first oligonucleotide library (O1b);
• a second oligonucleotide library (O3b) consisting of oligonucleotides (O3-x) each comprising a partial sequence forming the second partial sequence (S2) of the spacer element (S), at least one second variable codon triplet (T_{V2}) and a partial sequence corresponding to the defined second section (D2) of the nucleic acid library; and
• a second complementary oligonucleotide (04) or a second complementary oligonucleotide library, wherein the second complementary oligonucleotide (04) is substantially complementary to the oligonucleotides (O3-x) of the second oligonucleotide library (O3b) comprising a partial sequence (D2k) complementary to the second section (D2) of the second oligonucleotide library (O3b), at least one second complementary variable or substantially complementary variable codon triplet (T₂ₖ), a partial sequence (S2k) complementary to the second partial sequence (S2) of the spacer element (S) of the oligonucleotides (O3-x) of the second oligonucleotide library (O3b) and a subsequently arranged defined 3' overhang (U4);
OR
• a first oligonucleotide library (O1b) consisting of oligonucleotides (O1-x) each comprising a partial sequence corresponding to the defined first section (D1) of the nucleic acid library, at least one first variable codon triplet (T_{V1}) and a subsequently arranged first partial sequence (S1) of a spacer element (S);
• a first complementary oligonucleotide (O2) or a first substantially complementary oligonucleotide library, wherein the first complementary oligonucleotide (O2) is substantially complementary to the oligonucleotides (O1-x) of the first oligonucleotide library (O1b) comprising a defined 5' overhang, a partial sequence (S1) complementary to the first partial sequence (S1) of the spacer element (S) of the oligonucleotides (O1-x) of the first oligonucleotide library (O1b), at least one first complementary variable or substantially complementary variable codon triplet (Tvu) and a partial sequence (D1k) complementary to the first section (D1) of the oligonucleotides (O1-x) of the first oligonucleotide library (O1b);
• a second oligonucleotide library (O3b) consisting of oligonucleotides (O3-x) with a defined 5' overhang comprising a partial sequence forming the second partial sequence (S2) of the spacer element (S), at least one second variable codon triplet (T_{V2}) and a partial sequence corresponding to the defined second section (D2) of the nucleic acid library;
• a second complementary oligonucleotide (04) or a second substantially complementary oligonucleotide library, wherein the second complementary oligonucleotide (04) is substantially complementary to the oligonucleotides (O3-x) of the second oligonucleotide library (O3b) comprising a partial sequence (D2k) complementary to the second section (D2) of the second oligonucleotide library (O3b), at least one second complementary variable or substantially complementary variable codon triplet (T₂ₖ), and a partial sequence (S2k) complementary to the second partial sequence (S2) of the spacer element (S) of the oligonucleotides (O3-x) of the second oligonucleotide library (O3b);
wherein the following procedural steps are performed:
• hybridization of the oligonucleotides (O1-x) of the first oligonucleotide library (O1b) to the first substantially complementary oligonucleotide (O2) to generate a first double- stranded DNA library (O1xO2) and hybridization of the oligonucleotides (O3-x) of the second oligonucleotide library (O3b) to the second substantially complementary oligonucleotide (04) to generate a second double- stranded DNA library (O3x04);
• ligation of the first double- stranded DNA library (O1xO2) to the second doublestranded DNA library (O3x04), thereby producing a library of double- stranded DNA ligation products (LPx), the library of double- stranded DNA ligation products (LPx) comprising the following subsections: a defined first section (D1) of the nucleic acid library, at least one first variable codon triplet (T_{V1}), a spacer element (S), at least one second variable codon triplet (T_{V2}) and a defined second section (D2) of the nucleic acid library;
• removal of the spacer element (S) from the library of double- stranded DNA ligation products (LPx), thereby producing a library of double- stranded DNA-product (Px) comprising the following subsections: a defined first section (D1) of the nucleic acid library, at least one first variable codon triplet (T_{V1}), at least one second variable codon triplet (T_{V2}) and a defined second section (D2) of the nucleic acid library.

2. Method according to claim 1, wherein the at least one first variable codon triplet (Tvu) and the at least one second complementary variable codon triplet (Tvu) each consisting of three "universal- bases" (U) or a mixture of all four canonical nucleotides (A, T, C, G).

3. Method according to claim 1 or 2, wherein the defined 3' overhangs (U1, U4) of the oligonucleotides (O1-x) of the first oligonucleotide library (O1b) and the second complementary oligonucleotide (04) comprise the same number of nucleotides, wherein the nucleotides are at least substantially complementary to each other or wherein the defined 5' overhangs of the oligonucleotides (O2-x) of the second oligonucleotide library (O2b) and the first complementary oligonucleotide (O3) comprise the same number of nucleotides, wherein the nucleotides are at least substantially complementary to each other.

4. Method according to one of the previous claims, wherein the oligonucleotides (O1-x) of the first oligonucleotide library (O1b) and the second complementary oligonucleotide (04) each have defined 3' overhangs (U1, U4) that are at least substantially complementary to each other and wherein the first complementary oligonucleotide (O2) and the oligonucleotides (O3-x) of the second oligonucleotide library (O3b) each have defined 3' overhangs (U2, U3) that are each different in length and not complementary to one another and wherein the defined 3' overhangs (U) of the first complementary oligonucleotide (O2) and the oligonucleotides (O3-x) of the second oligonucleotide library (O3b) are each different in length and not complementary to the defined 3' overhangs (U1, U4) of the oligonucleotides (O1-x) of the first oligonucleotide library (O1b) and the second complementary oligonucleotide (04).

5. Method according to one of the claims 1 to 3, wherein the oligonucleotides (O3-x) of the second oligonucleotide library (O3b) and the first complementary oligonucleotide (O2) each comprising defined, at least substantially complementary 5' overhangs and wherein the second complementary oligonucleotide (04) and the oligonucleotides (O1-x) of the first oligonucleotide library (O1b) each have defined 5' overhangs that are each different in length and not complementary to each other and wherein the defined 5' overhangs of the second complementary oligonucleotide (04) and the oligonucleotides (O1-x) of the first oligonucleotide library (O1b) are each different in length and not complementary to the defined 5' overhangs of the oligonucleotides (O3-x) of the second oligonucleotide library (O3b) and the first complementary oligonucleotide (O2).

6. Method according to one of the previous claims, wherein the spacer element (S) comprises at least one recognition site (3) for at least one type IIS restrictions endonuclease (2), wherein the least one type IIS restrictions endonuclease (2) cuts the double- stranded DNA ligation product (LPx) 3' after the third nucleotide of the first variable codon triplet (T_{V1}) and / or 5' in front of the first nucleotide of the second variable codon triplet (T_{V2}).

7. Method according to one of the previous claims, wherein the first oligonucleotide library (O1b) comprises two adjoining or substantially adjoining variable codon triplets (T_{V1}, T_{V2}) and / or wherein the second oligonucleotide library (O3b) comprises two adjoining or substantially adjoining variable codon triplets (T_{V3}, T_{V4}) and wherein the first and / or the second oligonucleotide (O2, 04) each comprises two adjoining or substantially adjoining complementary variable codon triplets (Tvu) consisting of three "universal bases" (U) or consisting of a mixture of all four canonical nucleotides (A, T, C, G).

8. Method according to claim 7, wherein a nucleic acid sequence comprising three or four adjoining variable codon triplets (T_{V1} - T_{V4}) is produced from the two oligonucleotide libraries (O1b, O3b) and the two oligonucleotides (O2, 04) according to claim 7 by the procedural steps described in claim 1.

9. Method according to claim 6, wherein
• the library of double- stranded DNA ligation product (LPx) is integrated by ligation into a suitably prepared cloning vector (pK) using the defined 3' overhangs (U2, U3) of the first oligonucleotide (O2) and of the respective oligonucleotide (O3-x) of the second oligonucleotide library (O3b) and the corresponding overhangs (U2*; U3*) of the a suitably prepared cloning vector (pK), thereby producing a library of ligation product vector (pK-LPx);
• the library of ligation product vector (pK-LPx) is treated with at least one type IIS restrictions endonuclease (2a, 2b), wherein the spacer element (S) is excised;
• the library of cut ligation product vector (pK-LPx-S) is religated and cyclized forming a library of product vector (pK-Px);
• the library of product vector (pK-Px) comprising a double- stranded DNA product (Px) with the following partial sequences: a defined first section (D1) of the nucleic acid library, at least one first variable codon triplet (T_{V1}), at least one second variable codon triplet (T_{V2}) and a defined second section (D2) of the nucleic acid library.

10. Method according to claim 9, wherein a first library of product vector (pK-Px1) with a first variable nucleic acid sequence (T1) comprises at least two different selection markers (M1, M2), wherein a second library of product vector (pK-Px2) with a second variable nucleic acid sequence (T2) comprises at least two further different selection markers (M3, M4), wherein both libraries of product vector (pK-Px1, pK-Px2) are each cut at two locations by suitable restriction endonucleases and wherein the fragments are combined by religation, wherein the desired product vectors (pK-Px, pK-Px4) are specifically selected on the basis of a newly created selection marker combinations (M1/M4 or M3/ M2).

## Revendications

1. Procédé de préparation d'une séquence d'ADN pour une banque d'acides nucléiques comprenant une pluralité d'éléments,
dans lequel la séquence d'ADN comprend au moins trois régions,
dans lequel les première et troisième régions dans chaque élément présentent chacune des séquences définies (D1, D2) qui sont respectivement identiques dans l'ensemble des éléments de la banque d'acides nucléiques,
dans lequel une deuxième région intermédiaire comprend respectivement au moins deux triplets de codon variables voisins,
procédé dans lequel au moins deux banques d'oligonucléotides et au moins deux oligonucléotides simple brin ayant les caractéristiques suivantes sont utilisés:
• une première banque d'oligonucléotides (O1b) se composant d'oligonucléotides (O1-x) ayant chacun une séquence partielle qui correspond à la première région définie (D1) de la banque d'acides nucléiques, au moins un premier triplet de codon variable (T_{V1}), une première séquence partielle (S1) y contiguë d'un espaceur (S) et un surplomb 3' défini (U1),
• un premier oligonucléotide complémentaire (O2) ou une première banque d'oligonucléotides complémentaires, le premier oligonucléotide complémentaire (O2) étant dans une large mesure complémentaire des oligonucléotides (O1-x) de la première banque d'oligonucléotides (O1b), comprenant une séquence partielle (S1k) qui est réalisée de façon complémentaire de la première séquence partielle (S1) de l'espaceur (S) des oligonucléotides (O1-x) de la première banque d'oligonucléotides (O1b), au moins un premier triplet de codon complémentaire-variable ou bien dans une large mesure complémentaire-variable (Tvu) et une séquence partielle (D1k) qui est réalisée de façon complémentaire de la première région (D1) des oligonucléotides (O1-x) de la première banque d'oligonucléotides (O1b),
• une deuxième banque d'oligonucléotides (O3b) se composant d'oligonucléotides (O3-x) ayant une séquence partielle qui forme la deuxième séquence partielle (S2) de l'espaceur (S), au moins un deuxième triplet de codon variable (T_{V2}) et une séquence partielle qui correspond à la deuxième région définie (D2) de la banque d"acides nucléiques; et
• un deuxième oligonucléotide complémentaire (04) ou une deuxième banque d'oligonucléotides complémentaires, le deuxième oligonucléotide complémentaire (04) étant dans une large mesure complémentaire des oligonucléotides (O3-x) de la deuxième banque d'oligonucléotides (O3b), avec une séquence partielle (D2k) qui est réalisée de façon complémentaire de la deuxième région (D2) de la deuxième banque d'oligonucléotides (O3b), au moins un deuxième triplet de codon complémentaire-variable ou bien dans une large mesure complémentaire-variable (T₂ₖ), avec une séquence partielle (S₂ₖ) qui est réalisée de façon complémentaire de la deuxième séquence partielle (S2) de l'espaceur (S) des oligonucléotides (O3-x) de la deuxième banque d'oligonucléotides (O3b), et un surplomb 3' défini (U4) y contigu;
OU
• une première banque d'oligonucléotides (O1b) se composant d'oligonucléotides (O1-x) ayant chacun une séquence partielle qui correspond à la première région définie (D1) de la banque d'acides nucléiques, au moins un premier triplet de codon variable (T_{V1}) et une première séquence partielle (S1) y contiguë d'un espaceur (S),
• un premier oligonucléotide complémentaire (O2) ou une première banque d'oligonucléotides complémentaires, le premier oligonucléotide complémentaire (O2) étant dans une large mesure complémentaire des oligonucléotides (O1-x) de la première banque d'oligonucléotides (O1b), avec un surplomb 5' défini, avec une séquence partielle (S1k) qui est réalisée de façon complémentaire de la première séquence partielle (S1) de l'espaceur (S) des oligonucléotides (O1-x) de la première banque d'oligonucléotides (O1b), au moins un premier triplet de codon complémentaire-variable ou bien dans une large mesure complémentaire-variable (Tvu) et une séquence partielle (D1k) qui est réalisée de façon complémentaire de la première région (D1) des oligonucléotides (O1-x) de la première banque d'oligonucléotides (O1b),
• une deuxième banque d'oligonucléotides (O3b) se composant d'oligonucléotides (O3-x) avec un surplomb 5' défini, avec une séquence partielle qui forme la deuxième séquence partielle (S2) de l'espaceur (S), au moins un deuxième triplet de codon variable (T_{V2}) et une séquence partielle qui correspond à la deuxième région définie (D2) de la banque d'acides nucléiques;
• un deuxième oligonucléotide complémentaire (04) ou une deuxième banque d'oligonucléotides complémentaires, le deuxième oligonucléotide complémentaire (04) étant dans une large mesure complémentaire des oligonucléotides (O3-x) de la deuxième banque d'oligonucléotides (O3b), avec une séquence partielle (D2k) qui est réalisée de façon complémentaire de la deuxième région (D2) de la deuxième banque d'oligonucléotides (O3b), au moins un deuxième triplet de codon complémentaire-variable ou bien dans une large mesure complémentaire-variable (T₂ₖ) et avec une séquence partielle (S₂ₖ) qui est réalisée de façon complémentaire de la deuxième séquence partielle (S2) de l'espaceur (S) des oligonucléotides (O3-x) de la deuxième banque d'oligonucléotides (O3b);
dans lequel les étapes de procédé suivantes sont mises en oeuvre:
• l'hybridation des oligonucléotides (O1-x) de la première banque d'oligonucléotides (O1b) avec le premier oligonucléotide dans une large mesure complémentaire (O2) pour obtenir une première banque d'ADN double brin (O1xO2) ainsi que l'hybridation des oligonucléotides (O3-x) de la deuxième banque d'oligonucléotides (O3b) avec le deuxième oligonucléotide dans une large mesure complémentaire (04) pour obtenir une deuxième banque d'ADN double brin (O3x04);
• la ligature de la première banque d'ADN double brin (O1 xO2) avec la deuxième banque d'ADN double brin (O3x04) en formant une banque de produits de ligature d'ADN double brin (L_{PX}) qui comprennent les sections suivantes: la première région définie (D1) de la banque d'acides nucléiques, au moins un premier triplet de codon variable (T_{V1}), la séquence espaceur (S), au moins un deuxième triplet de codon variable (T_{V2}) et la deuxième région définie (D2) de la banque d'acides nucléiques;
• l'élimination de la séquence espaceur (S) de la banque de produit de ligature d'ADN double brin (LPx) en formant une banque de produit d'ADN double brin (Px) qui comprend les sections suivantes: la première région définie (D1) de la banque d'acides nucléiques, au moins un premier triplet de codon variable (T_{V1}), au moins un deuxième triplet de codon variable (T_{V2}) et la deuxième région définie (D2) de la banque d'acides nucléiques.

2. Procédé selon la revendication 1, dans lequel ledit au moins un premier triplet de codon complémentaire-variable (Tvu) et ledit au moins un deuxième triplet de codon complémentaire-variable (Tvu) comprennent chacun trois "bases universelles" (U) ou un mélange de tous les quatre nucléotides canoniques (A, T, C, G).

3. Procédé selon la revendication 1 ou 2, dans lequel les surplombs 3' définis (U1, U4) des oligonucléotides (O1-x) de la première banque d'oligonucléotides (O1b) et du deuxième oligonucléotide complémentaire (04) comprennent le même nombre de nucléotides et sont au moins dans une large mesure complémentaires les uns des autres, ou dans lequel les surplombs 5' définis des oligonucléotides (O2-x) de la deuxième banque d'oligonucléotides (O2b) et du premier oligonucléotide complémentaire (O3) comprennent le même nombre de nucléotides et sont au moins dans une large mesure complémentaires les uns des autres.

4. Procédé selon l'une quelconque des revendications précédentes, dans lequel les oligonucléotides (O1-x) de la première banque d'oligonucléotides (O1b) et le deuxième oligonucléotide complémentaire (04) présentent chacun des surplombs 3' (U1, U4) définis et au moins dans une large mesure complémentaires les uns des autres, et dans lequel le premier oligonucléotide complémentaire (O2) et les oligonucléotides (O3-x) de la deuxième banque d'oligonucléotides (O3b) présentent chacun des surplombs 3' définis (U2, U3) qui présentent chacun une longueur différente et ne sont pas réalisés de façon complémentaire les uns des autres, et dans lequel les surplombs 3' définis (U) du premier oligonucléotide complémentaire (O2) et des oligonucléotides (O3-x) de la deuxième banque d'oligonucléotides (O3b) ont chacun une longueur différente et ne sont pas réalisés de façon complémentaire des surplombs 3' définis (U1, U4) des oligonucléotides (O1-x) de la première banque d'oligonucléotides (O1b) et du deuxième oligonucléotide complémentaire (04).

5. Procédé selon l'une quelconque des revendications 1 à 3, dans lequel les oligonucléotides (O3-x) de la deuxième banque d'oligonucléotides (O3b) et le premier oligonucléotide complémentaire (O2) présentent chacun des surplombs 5' définis et au moins dans une large mesure complémentaires les uns des autres, et dans lequel le deuxième oligonucléotide complémentaire (04) et les oligonucléotides (O1-x) de la première banque d'oligonucléotides (O1b) présentent chacun des surplombs 5' définis qui présentent chacun une longueur différente et ne sont pas réalisés de façon complémentaire les uns des autres, et dans lequel les surplombs 5' définis du deuxième oligonucléotide complémentaire (04) et des oligonucléotides (O1-x) de la première banque d'oligonucléotides (O1b) ont chacun une longueur différente et ne sont pas réalisés de façon complémentaire des surplombs 5' définis des oligonucléotides (O3-x) de la deuxième banque d'oligonucléotides (O3b) et du premier oligonucléotide complémentaire (O2).

6. Procédé selon l'une quelconque des revendications précédentes, dans lequel la séquence espaceur (S) comprend au moins une séquence de reconnaissance (3) pour au moins une endonucléase de restriction de type IIS (2), dans lequel ladite au moins une endonucléase de restriction de type IIS (2) coupe le produit de ligature d'ADN double brin (LPx) 3' derrière le troisième nucléotide du premier triplet de codon variable (T_{V1}) et / ou 5' devant le premier nucléotide du deuxième triplet de codon variable (T_{V2}).

7. Procédé selon l'une quelconque des revendications précédentes, dans lequel la première banque d'oligonucléotides (O1b) comprend deux triplets de codon variables voisins ou dans une large mesure voisins (T_{V1}, T_{V2}) et / ou la deuxième banque d'oligonucléotides (O3b) comprend deux triplets de codon variables voisins ou dans une large mesure voisins (T_{V3}, T_{V4}), et dans lequel le premier et / ou le deuxième oligonucléotide (O2, 04) comprennent chacun deux triplets de codon complémentaires-variables voisins ou dans une large mesure voisins (Tvu) chacun en trois "bases universelles" (U) ou en un mélange de tous les quatre nucléotides canoniques (A, T, C, G).

8. Procédé selon la revendication 7, dans lequel, par les étapes de procédé décrites dans la revendication 1, une séquence d'acide nucléique ayant trois ou quatre triplets de codon variables voisins (T_{V1} - T_{V4}) est produite à partir des deux banques d'oligonucléotides (O1b, O3b) et des deux oligonucléotides (O2, 04) selon la revendication 7.

9. Procédé selon la revendication 6, dans lequel
• la banque de produit de ligature d'ADN double brin (LPx) est intégré, via les surplombs 3' définis (U2, U3) du premier oligonucléotide (O2) et de l'oligonucléotide respectif (O3-x) de la deuxième banque d'oligonucléotides (O3b), par ligature à un vecteur de clonage (pK) préparé de manière correspondante, avec des surplombs correspondants (U2*; U3*), et une banque de vecteur de produit de ligature (pK-LPx) est formée;
• la banque de vecteur de produit de ligature (pK-LPx) est traitée par au moins une endonucléase de restriction de type IIS (2a, 2b), la séquence espaceur (S) étant découpée;
• la banque de vecteur de produit de ligature coupé (pK-LPx-S) est religaturée et est cyclisée en la banque de vecteur de produit (pK-Px);
• la banque de vecteur de produit (pK-Px) comprend un produit d'ADN double brin (Px) ayant les sections suivantes: la première région définie (D1) de la banque d'acides nucléiques, le premier triplet de codon variable (T_{V1}), le deuxième triplet de codon variable (T_{V2}) et la deuxième région définie (D2) de la banque d'acides nucléiques.

10. Procédé selon la revendication 9, dans lequel une première banque de vecteur de produit (pK-Px1) avec une première séquence d'acide nucléique variable (T1) comprend au moins deux marqueurs de sélection différents (M1, M2), dans lequel une deuxième banque de vecteur de produit (pK-Px2) avec une deuxième séquence d'acide nucléique variable (T2) comprend au moins deux autres marqueurs de sélection différents (M3, M4), les deux banques de vecteur de produit (pK-Px1, pK-Px2) étant coupées chacune à deux endroits au moyen d'endonucléases de restriction appropriées et les fragments étant combinés entre eux par religature, dans lequel les vecteurs de produit souhaités (pK-Px, pK-Px4) sont sélectionnés de manière ciblée à partir de la combinaison de marqueurs de sélection nouvellement créée (M1/M4 ou bien M3/M2).
